(19) Europäisches Patentamt
European Patent Office
Office européen des brevets

(11) EP 2 356 935 B1

# (12) EUROPEAN PATENT SPECIFICATION

(45) Date of publication and mention of the grant of the patent:
**08.03.2017 Bulletin 2017/10**

(21) Application number: **09826183.7**

(22) Date of filing: **16.11.2009**

(51) Int Cl.:
*A61B 1/04* (2006.01) *A61B 1/00* (2006.01)
*A61B 1/06* (2006.01) *G02B 23/24* (2006.01)

(86) International application number:
**PCT/JP2009/069435**

(87) International publication number:
**WO 2010/055938 (20.05.2010 Gazette 2010/20)**

(54) **IMAGE-PROCESSING SYSTEM, IMAGING DEVICE, RECEIVING DEVICE, AND IMAGE DISPLAY DEVICE**

BILDVERARBEITUNGSSYSTEM, BILDGEBUNGSVORRICHTUNG, EMPFANGSVORRICHTUNG UND BILDANZEIGEVORRICHTUNG

SYSTÈME DE TRAITEMENT D'IMAGE ET DISPOSITIF D'IMAGERIE, DISPOSITIF DE RÉCEPTION ET DISPOSITIF D'AFFICHAGE D'IMAGE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **17.11.2008 JP 2008293834**

(43) Date of publication of application:
**17.08.2011 Bulletin 2011/33**

(73) Proprietor: **Olympus Corporation**
**Tokyo 192-8507 (JP)**

(72) Inventors:
- **TAMURA, Kazuaki**
  **Hachioji-shi,**
  **Tokyo 192-8507 (JP)**
- **MORI, Takeshi**
  **Hachioji-shi,**
  **Tokyo 192-8507 (JP)**
- **UCHIYAMA, Akio**
  **Hachioji-shi,**
  **Tokyo 192-8507 (JP)**

(74) Representative: **Gunzelmann, Rainer et al**
**Wuesthoff & Wuesthoff**
**Patentanwälte PartG mbB**
**Schweigerstraße 2**
**81541 München (DE)**

(56) References cited:
**WO-A2-2008/095183**
**JP-A- 2005 074 034**
**JP-A- 2005 074 034**
**JP-A- 2006 075 331**
**JP-A- 2006 122 502**
**JP-A- 2007 021 039**
**JP-A- 2007 215 927**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Field

The present invention relates to a capsule endoscope system for performing an appropriate generation of ordinary images such as white color images and spectral images consisting of specific color components.

### Background

In recent years, capsule body-insertable apparatuses (for example, capsule endoscopes) provided with an imaging function and a radio communication function have been proposed in the field of endoscope and body-insertable systems to acquire intra-subject images using such capsule endoscopes have been developed. To make intra-subject observations (examinations), for example, after being swallowed through the mouth of a subject, a capsule endoscope moves through a body cavity, for example, inside organs such as stomach and small intestine following peristaltic movement and also functions to capture intra-subject images at intervals of, for example, 0.5 s before being naturally discharged.

While a capsule endoscope moves through inside a subject, images captured by the capsule endoscope are received by an external image display device via an antenna arranged on the body surface of the subject. The image display device has the radio communication function for the capsule endoscope and a memory function of images and successively stores an image received from the in-vivo capsule endoscope into a memory. A doctor or nurse can make intra-subject observations (examinations) and make a diagnosis by displaying images, that is, images inside an alimentary canal of the subject accumulated in such an image display device in a display.

Patent Literature 1 describes an in-vivo imaging device in which a plurality of individual light sources and a plurality of individual optical sensors are arranged and the operation and gain of light sources are controlled based on the quantity of light sensed by optical sensors of light reflected by an object when light sources operate.

### Citation List

#### Patent Literature

Patent Literature 1: Japanese Laid-open Patent Publication No. 2006-247404

JP 2007 215927 A concerns a technique to prepare pseudo color images for easily visually recognizing an area of a low fluorescent light emitting amount and an area of a high fluorescent light emitting amount. For this, an endoscope processor is provided with an AGC circuit and a pseudo color image computing circuit. A light source unit alternately irradiates an object with reference light and excitation light. When the reference light is radiated, an imaging element generates reference light image signals. When the excitation light is radiated, the imaging element generates fluorescent image signals. On the basis of the reference light image signals, the AGC circuit normalizes the luminance components of the fluorescent image signals. On the basis of the reference light image signals and the normalized fluorescent image signals, the pseudo color image computing circuit calculates relative luminance. The pseudo color image computing circuit prepares the pseudo color images which have the colored areas of relatively low luminance and a relatively high luminance.

### Summary

#### Technical Problem

In the image generation system including an imaging device such as a capsule endoscope, the captured images may not have a constant brightness. Accordingly, there is a problem in that it is different to generate image data itself and perform processing on the generated image data with stability.

The present invention has been made in view of the above problem, and an object of the invention is to provide a capsule endoscope system capable of generating image data itself and performing processing on the generated image data with stability.

#### Solution to Problem

To solve the problem and achieve the object, the system according to the present invention includes the features of claim 1

The dependent claims concern further advantageous aspects of the invention.

Advantageous Effects of Invention

According to the present invention, since appropriate control in accordance with image brightness information is exercised, it is possible to provide a capsule endoscope system capable of generating image data itself and performing processing on the generated image data with stability.

Brief Description of Drawings

FIG. 1 is a block diagram showing an outline configuration of an image processing system according to an embodiment not forming part of the present invention.
FIG. 2 is a diagram showing an overall outline configuration of a capsule endoscope system according to a second embodiment of the present invention.
FIG. 3 is a plan view near an imaging unit and an illuminating unit of a capsule endoscope according to the second embodiment of the present invention.
FIG. 4 is a sectional view near the imaging unit and the illuminating unit of the capsule endoscope according to the second embodiment of the present invention.
FIG. 5 is a diagram showing wavelength dependency of a light absorption characteristic level of blood.
FIG. 6 is a schematic view showing a relationship between transmission and reflection of light with regard to an inner wall of a body cavity and blood vessel.
FIG. 7 is a block diagram showing the configuration of the capsule endoscope according to the second embodiment of the present invention.
FIG. 8 is a flow chart showing an observation mode control processing procedure by an observation mode controller inside the capsule endoscope according to the second embodiment of the present invention.
FIG. 9 is a timing chart showing an example of observation mode control processing by the observation mode controller inside the capsule endoscope according to the second embodiment of the present invention.
FIG. 10 is a plan view near the imaging unit and the illuminating unit of the capsule endoscope according to a third embodiment of the present invention.
FIG. 11 is a sectional view near the imaging unit and the illuminating unit of the capsule endoscope according to the third embodiment of the present invention.
FIG. 12 is a flow chart showing the observation mode control processing procedure by the observation mode controller inside the capsule endoscope according to the third embodiment of the present invention.
FIG. 13 is a flow chart showing the observation mode control processing procedure by the observation mode controller inside the capsule endoscope according to a fourth embodiment of the present invention.
FIG. 14 is a flow chart showing the observation mode control processing procedure by the observation mode controller inside the capsule endoscope according to a fifth embodiment of the present invention.
FIG. 15 is a block diagram showing the configuration of the capsule endoscope according to a sixth embodiment of the present invention.
FIG. 16 is a flow chart showing a light emission quantity adjustment processing procedure by a light emission quantity adjustment unit shown in FIG. 15.
FIG. 17 is a flow chart showing the light emission quantity adjustment processing procedure by the light emission quantity adjustment unit of the capsule endoscope according to a seventh embodiment of the present invention.
FIG. 18 is a block diagram showing the configuration of a receiving device according to an eighth embodiment of the present invention.
FIG. 19 is a flow chart showing a brightness adjustment processing procedure by a brightness adjustment unit shown in FIG. 18.
FIG. 20 is a block diagram showing the configuration of the capsule endoscope according to a ninth embodiment of the present invention.
FIG. 21 is a flow chart showing an outline operation of the capsule endoscope according to the ninth embodiment of the present invention.
FIG. 22 is a flow chart showing the outline operation of the receiving device 3 according to the ninth embodiment of the present invention.
FIG. 23 is a flow chart showing the outline operation of an image display device according to a tenth embodiment of the present invention.
FIG. 24 is a flow chart showing the outline operation of an example of an image processing function (motion detection function) executed by the image display device 4 according to the tenth embodiment of the present invention.

FIG. 25 is a flow chart showing the outline operation of another example of the image processing function (red detection function) executed by the image display device according to the tenth embodiment of the present invention.

FIG. 26 is a block diagram showing the configuration of the capsule endoscope according to an eleventh embodiment of the present invention.

FIG. 27 is a flow chart showing the outline operation of the capsule endoscope according to the eleventh embodiment of the present invention.

FIG. 28 is a flow chart showing the outline operation of the receiving device according to the eleventh embodiment of the present invention.

FIG. 29 is a flow chart showing the outline operation of the image display device according to the eleventh embodiment of the present invention.

Description of Embodiments

Preferred embodiments of the present invention will be described in detail with reference to drawings. All embodiments shown below can be combined in all their configurations or a portion thereof when appropriate.

First embodiment not forming part of the present invention

First, before describing a capsule endoscope system according to the first embodiment, the configuration of an image processing system to be a construction concept of the capsule endoscope system will be described in detail using drawings. FIG. 1 is a block diagram showing an outline configuration of an image processing system according to the present embodiment. As shown in FIG. 1, an image processing system 100 according to the present embodiment roughly includes an image generation unit 101, a display unit 102, a brightness detection unit 106, and a control unit 107. The image generation unit 101 is a unit that has at least two observation modes that alternately or continuously generate different types of images, for example, images consisting of combinations of color components (for example, ordinary light images and special light images to be described later) and selects one of the observation modes to generate images in the selected observation mode. The image generation unit 101 includes an imaging unit 104 that captures an image of an object, an illuminating unit 103 that illuminate the object during imaging, and an image processing unit 105 that performs predetermined image processing on image data obtained by imaging. The brightness detection unit 106 detects information indicating brightness (hereinafter, referred to simply as brightness information) of an image obtained by imaging of the imaging unit 104 in one observation mode. The control unit 107 controls generation of an image in the other observation mode by the image generation unit 101 by controlling at least one of the imaging unit 104, the illuminating unit 103, and the image processing unit 105 of the image generation unit 101 based on brightness information detected by the brightness detection unit 106.

As brightness information in the present embodiment, all information indicating image brightness, for example, an exposure time when the imaging unit 104 captures an image, average luminance of images acquired by the imaging unit 104, and an integral value (also called a light exposure) of signal strength of pixels contained in a predetermined region of an acquired image can be used as brightness information.

The control unit 107 exercises control such as determining the light emission quantity (power) or light emission time of the illuminating unit 103 and selecting the type of a driven light source based on brightness information detected by the brightness detection unit 106. The control unit 107 also exercises control such as determining the exposure time by the imaging unit 104 and selecting the type (one or more of R, G, and B) of pixels of an image signal to be read similarly based on the detected brightness information. Further, the control unit 107 exercises control such as changing various parameters in image processing by the image processing unit 105 and selecting the image processing function to be executed similarly based on the detected brightness information.

In the present embodiment, as described above, appropriate control in accordance with image brightness is exercised by controlling the imaging unit 104, the illuminating unit 103, or the image processing unit 105 in the image generation unit 101 based on the acquired image brightness information so that it becomes possible to generate image data itself and perform processing on the generated image data with stability.

Second embodiment

Next, as an image processing system according to the second embodiment of the present invention, a capsule endoscope system using a capsule endoscope as an imaging device is taken as an example. The capsule endoscope system according to the present embodiment is an embodiment of the image processing system according to the first embodiment described above and the concept thereof is contained in the concept of the image processing system.

FIG. 2 is a schematic diagram showing the configuration of a capsule endoscope system according to the second embodiment of the present invention. As shown in FIG. 2, the capsule endoscope system according to the second embodiment includes a capsule endoscope 2 as an imaging device to capture an in-vivo image of a subject 1, a receiving device 3 that receives an image signal transmitted from the capsule endoscope 2 by radio, an image display device 4 that displays the in-vivo image captured by the capsule endoscope 2, a portable recording medium 5 that exchanges data between the receiving device 3 and the image display device 4.

The capsule endoscope 2 is equipped with the imaging function and radio communication function inside a capsule casing. The capsule endoscope 2 is inserted into an organ of the subject 1 through ingestion intake or the like and then, successively captures an in-vivo image of the subject 1 at predetermined intervals (for example, at intervals of 0.5 s) while moving through inside the organ of the subject 1 due to peristaltic movement or the like. More specifically, the capsule endoscope 2 alternately captures an ordinary image using white light (ordinary light observation) and a spectral image generated by using special light consisting of specific color components of blue and green (special light observation) such as a sharp blood vessel image of the inner wall of body cavity including a plurality of repetitions of each. The capsule endoscope 2 transmits an image signal of in-vivo images of the subject 1 captured in this manner to the outside receiving device 3 by radio. The capsule endoscope 2 successively repeats the imaging operation and radio transmission operation of such in-vivo images in a period between insertion into organs of the subject 1 and the discharge out of the subject 1.

The receiving device 3 is equipped with a plurality of receiving antennas 3a to 3h arranged, for example, on a body surface of the subject 1 in a distributed fashion and receives a radio signal from the capsule endoscope 2 inside the subject 1 via at least one of the plurality of receiving antennas 3a to 3h. The receiving device 3 extracts an image signal from the radio signal output from the capsule endoscope 2 to acquire image data of in-vivo images contained in the extracted image signal.

The receiving device 3 also performs various kinds of image processing on the acquired image data and stores a group of the image-processed in-vivo images in the recording medium 5 inserted in advance. The receiving device 3 also associates each image of the group of in-vivo images with time data such as the imaging time or receiving time.

The receiving antennas 3a to 3h of the receiving device 3 may be arranged, as shown in FIG. 2, on the body surface of the subject 1 or on a jacket put on by the subject 1. The number of receiving antennas of the receiving device 3 may be equal to 1 or more and is not particularly limited to eight.

The image display device 4 is configured like a workstation that captures various kinds of data such as a group of in-vivo images of the subject 1 via the recording medium 5 and displays various kinds of data of the captured group of in-vivo images or the like. More specifically, after the recording medium 5 removed from the receiving device 3 being inserted into, the image display device 4 captures saved data of the recording medium 5 to acquire various kinds of data such as a group of in-vivo images of the subject 1. The image display device 4 has a function to display acquired in-vivo images in a display. A diagnosis is made based on the image display by the image display device 4.

The recording medium 5 is a portable recording medium to exchange data between the receiving device 3 and the image display device 4 described above. The recording medium 5 is structured to be removable from the receiving device 3 and the image display device 4 and to be able to output and record data when inserted into one of the receiving device 3 and the image display device 4. More specifically, when inserted into the receiving device 3, the recording medium 5 records a group of in-vivo images processed by the receiving device 3 and time data of each image.

The capsule endoscope 2 contains various functions inside a capsule casing 21, one end thereof is covered with a dome-shaped transparent cover 20, and the illuminating unit and imaging unit are arranged on the one end side. As shown in FIGS. 3 and 4, a lens barrel 24 is provided in a center section of a substrate 23 in a disc shape and an optical lens 13 for which a cylinder axis of the capsule casing 21 becomes the optical axis and an imaging element 14 are provided inside the lens barrel 24. Ordinary light sources 10a to 10c (10) realized by a white LED emitting white light and special light sources 11a to 11c (11) emitting light in a waveband having a peak near 415 nm (blue) and light in a waveband having a peak near 540 nm (green) are arranged alternately in different positions in an annular shape on the circumference side of the substrate 23. The special light source 11 is of dual wavelength emission type constructed by coating an LED chip emitting light of 415 nm with a phosphor emitting light of 540 nm. Each of the light sources 10 and 11 has approximately the same luminous intensity distribution characteristics. A transparent fixing member 12 is provided on each of the light sources 10 and 11. The imaging element 14 is realized by CCD of ordinary Bayer array or the like.

A special light observation using the special light source 11 will be described. First, as shown in FIG. 5, the light absorption characteristic level of blood is low except a peak L1 at 415 nm (blue) and a peak at 540 nm (green). As shown in FIG. 6, the inner wall of the body cavity has capillaries 43 present in a surface layer of mucosa 40 and further thick blood vessels 44 present in a deep part of mucosa 41. Light 30B of 415 nm (blue) irradiating the inner wall of the body cavity has a short wavelength and thus does not penetrate deep into tissues and instead, is absorbed by the capillaries 43 due to light absorption characteristics of blood described above. Light 30G of 540 nm (green) has a longer wavelength than blue and thus penetrates to the deep part of mucosa 41 and is absorbed by the thick blood vessels 44 due to light absorption characteristics of blood described above. On the other hand, red light 30R penetrates to internal tissues 42 and is mostly reflected as scattered light. Thus, contrast information of an image of blood vessels such as the capillaries 43 and the thick blood vessels 44 can be obtained by providing receiving sensitivity of only 415 nm (blue) and 540 nm (green).

Therefore, in the special light observation, contrast information of the blood vessel can be obtained and also a spectral image, which is a blood vessel image, can be obtained by irradiating an object with light having wavelengths of blue and green and using an imaging element having sensitivity characteristics of wavelengths of blue and green.

FIG. 7 is a block diagram showing a detailed configuration of the capsule endoscope 2. As shown in FIG. 7, the capsule endoscope 2 includes an illuminating unit 51 that emits illuminating light of an object, an imaging unit 52 that images the object by receiving reflected light from the object, a state detection unit 53 through which the capsule detects states inside and outside the capsule, a system controller 54 that controls the whole capsule endoscope 2, a transmitting circuit 55 that transmits information such as image data captured by the imaging unit 52 to the outside of the capsule endoscope 2, particularly out of the subject via a transmitting antenna 56, and a power supply circuit 57 supplies power to various components under the control of the system controller 54.

The illuminating unit 51 includes the ordinary light source 10 and the special light source 11 described above and a light source control circuit 61 that drives and controls the ordinary light source 10 and the special light source 11. If the same current is supplied to the ordinary light source 10 and the special light source 11, the special light source 11 emits special light whose quantity of light is smaller than that of ordinary light. The imaging unit 52 includes the above imaging element 14 and an imaging element control circuit 62 that drives and controls the imaging element 14. Further, the state detection unit 53 includes a sensor unit 63 and a sensor unit control circuit 64 that drives and controls the sensor unit 63. The sensor unit 63 is at least realized by various sensors capable of detecting whether the capsule endoscope 2 is in a liquid such as water (whether in a liquid or a gas) inside the subject 1.

The system controller 54 includes an exposure time measuring unit 71 and an observation mode controller 72. The exposure time measuring unit 71 measures the exposure time of at least an ordinary light observation as brightness information. The observation mode controller 72, on the other hand, controls the operation of an ordinary light observation mode corresponding to a first observation mode for capturing an ordinary light image and a special light observation mode corresponding to a second imaging mode for capturing a special light image based on exposure time information measured by the exposure time measuring unit 71.

The observation mode control processing procedure by the observation mode controller 72 will be described with reference to FIGS. 8 and 9. As shown in FIG. 8, the observation mode controller 72 first emits ordinary light of a preset quantity of light from the ordinary light source 10 (step S101). Then, the observation mode controller 72 acquires an ordinary light image by capturing the image through the imaging unit 52 (step S102). The observation mode controller 72 transmits the ordinary light image to the receiving device 3 outside the subject via the transmitting circuit 55 and the transmitting antenna 56 as data (step S103). Then, the observation mode controller 72 determines whether the observation mode is the special light observation mode (step S104). If the observation mode is not the special light observation mode (step S104, No), the observation mode controller 72 proceeds to step S201 to continue the ordinary light observation mode. On the other hand, if the observation mode is the special light observation mode (step S104, Yes), the observation mode controller 72 further determines whether the exposure time of ordinary light is successively equal to a specified value or more based on the measurement result of the exposure time measuring unit 71 (step S105). If the exposure time is successively equal to the specified value or more (step S105, Yes), the observation mode controller 72 proceeds to step S101 to make an ordinary light observation by maintaining the ordinary light observation mode.

On the other hand, if the exposure time of ordinary light is not successively equal to the specified value or more (step S105, No), the observation mode controller 72 causes the special light source 11 to emit special light (step S106) and proceeds to step S102 to acquire a special light image capturing the image through the imaging unit 52. That is, the observation mode controller 72 causes the imaging unit 52 to perform an operation in special light observation mode.

Namely, when a special light observation is made in a preset alternating order, if the last exposure time and the exposure time before the last exposure are both equal to a specified value or more when a ordinary light observation is made, in other words, the quantity of reflected light of ordinary light is small, the observation mode controller 72 makes an ordinary light observation, instead of a special light observation, because a special light image having sufficient brightness cannot be obtained since the quantity of reflected light is small even when the special light observation is performed.

FIG. 9 is a timing chart showing operation control of concrete observation modes by the observation mode controller 72. FIG. 9 shows a case where the ordinary light observation and special light observation are made at time intervals of $\Delta T1$. A time $\Delta T2$ is an exposure time, a time $\Delta T3$ is a specified value, and a time tmax is a maximum exposure time setting value. In FIG. 9, as shown in an upper part, the ordinary light observation and special light observation are alternately made like ordinary light observation M11 $\rightarrow$ special light observation M21 $\rightarrow$ ordinary light observation M12 $\rightarrow$ special light observation M22 $\rightarrow$ ordinary light observation M13 in an initial time zone. If a special light observation should be made after the ordinary light observation M13, the exposure time $\Delta T2$ of the ordinary light observation M12 and that of the ordinary light observation M13 are both equal to the specified value $\Delta T3$ or more and thus, successively equal to the specified value $\Delta T3$ or more and thus, the observation mode controller 72 exercises operation control to make an ordinary light observation M14, instead of the special light observation, in the time of the special light observation. Then, an ordinary light observation M15 is made in the time zone in which an ordinary light observation is made and when the next special light observation should be made, a special light observation M23 is made because the exposure time ΔT2 during the ordinary light observation M15 is less than the specified value ΔT3 and is not successively equal to the specified value ΔT3 or more.

Thus, while the ordinary light observation is always made in a time zone in which the ordinary light observation and special light observation are alternately made, if the exposure time during ordinary light observation is successively equal to the specified value ΔT3 or more, a special light observation immediately thereafter is not made and instead, an ordinary light observation is made. Accordingly, an ordinary light image with sufficient brightness can be obtained, instead of a special light image without sufficient brightness, leading to efficient use of power.

Third embodiment

Next, as an image processing system according to the third embodiment of the present invention, a capsule endoscope system using a capsule endoscope as an imaging device is taken as an example. The capsule endoscope system according to the present embodiment is, like the capsule endoscope system according to the second embodiment, an embodiment of the image processing system according to the first embodiment described above and the concept thereof is contained in the concept of the image processing system.

In the third embodiment, the special light source 11 includes a pair of wide-directivity special light sources 111 (111a to 111c) having wide directivity with regard to the optical axis of the imaging element 14 and narrow-directivity special light sources 112 (112a to 112c) having narrow directivity. As shown in FIGS. 10 and 11, the wide-directivity special light source 111 and the narrow-directivity special light source 112 are arranged as a pair in an annular shape and the wide-directivity special light source 111 is arranged on an inner circumference of the narrow-directivity special light source 112. With the narrow-directivity special light sources 112 being arranged on the inner circumference of the wide-directivity special light sources 111, light from the wide-directivity special light sources 111 can be prevented from directly entering the imaging element 14 so that flat light can irradiate a wide region.

FIG. 12 is a flow chart showing the observation mode control processing procedure by the observation mode controller according to the third embodiment of the present invention. In FIG. 12, the observation mode controller 72 performs processing similar to steps S101 to S105 shown in FIG. 8 and in step S205 corresponding to step S105, determines whether the exposure time of ordinary light is successively equal to the specified value or more.

Then, if the exposure time of ordinary light is not successively equal to the specified value or more (step S205, No), the observation mode controller 72 causes the narrow-directivity special light sources 112 and the wide-directivity special light sources 111 to emit light (step S206) before proceeding to step S202 to cause an operation in special light observation mode.

On the other hand, if the exposure time of ordinary light is successively equal to the specified value or more (step S205, Yes), the observation mode controller 72 further determines whether the capsule endoscope 2 is in a liquid based on a detection result of the sensor unit 63 (step S207). If the capsule endoscope 2 is not in a liquid (step S207, No), the capsule endoscope 2 is in a gas and the observation mode controller 72 proceeds to step S201 to cause an ordinary light observation in this special light observation period. On the other hand, if the capsule endoscope 2 is in a liquid (step S207, Yes), the observation mode controller 72 causes only the wide-directivity special light sources 111 to emit light (step S208) before proceeding to step S202 to cause a special light observation. In this case, wide-directivity light irradiates so that a special light image of surroundings of an object close to the capsule endoscope 2 can be obtained.

Fourth embodiment

In the above second and third embodiments, it is assumed that the exposure time measuring unit 71 measures the exposure time of the imaging unit 52, but the present invention is not limited to this and measurements may be made by associating the light emission quantity of the ordinary light sources 10 and 110 with the exposure time. In this case, instead of step S105 in the flow chart shown in FIG. 8, as shown in step S305 in FIG. 13, whether the light emission quantity of ordinary light is successively equal to the specified value or more may be determined by the observation mode controller 72.

Fifth embodiment

In the above second to fourth embodiments, the observation mode controller 72 performs processing to determine whether to make a special light observation or to replace a special light observation with an ordinary light observation without making the special light observation, but the present invention is not limited to this and, for example, as shown in FIG. 14, if the light emission quantity of ordinary light is successively equal to the specified value or more after determination processing in step S405 corresponding to step S305, a special light observation is made after increasing the light emission quantity of the special light sources 11, 111, and 112 (step S406) and if the light emission quantity of ordinary light is not successively equal to the specified value or more (step S405, No), a special light observation is made after bringing the light emission quantity of the special light sources 11, 111, and 112 back to the initial value (step S407). By exercising control of the light emission quantity in this manner, observation curbing power consumption for the special light observation can be made.

Sixth embodiment

Next, as an image processing system according to the sixth embodiment of the present invention, a capsule endoscope system using a capsule endoscope as an imaging device is taken as an example. The capsule endoscope system according to the present embodiment is, like the capsule endoscope system according to any of the second to fifth embodiments, an embodiment of the image processing system according to the first embodiment described above and the concept thereof is contained in the concept of the image processing system.

In the sixth embodiment, as shown in FIG. 15, the capsule endoscope 2 is provided with the system controller 54 that adjusts brightness of each of ordinary light images and special light images obtained by individually adjusting the light emission quantity of the ordinary light sources 10 and the special light sources 11 to appropriate brightness.

The system controller 54 includes a light emission quantity adjustment unit 171 that makes light emission quantity adjustments of the ordinary light sources 10 and the special light sources 11 corresponding to each of ordinary light images and special light images. The system controller 54 also includes an observation mode controller 172 that exercises mode control such as switching each observation mode to capture ordinary light images and special light images.

The light emission quantity adjustment processing procedure by the light emission quantity adjustment unit 171 will be described with reference FIG. 16. First, the light emission quantity adjustment unit 171 determines whether the currently captured image is an ordinary light image based on control content of the observation mode controller 172 (step S501). If the image is an ordinary light image (step S501, Yes), the light emission quantity adjustment unit 171 adds up values of all pixels (R, G, B) within a predetermined range of the ordinary light image obtained last time (step S502). Then, the light emission quantity adjustment unit 171 determines whether the added value is within an appropriate range, that is, the image has appropriate brightness (step S503). If the added value is not within the appropriate range (step S503, No), the light emission quantity adjustment unit 171 makes light emission quantity adjustments of the ordinary light sources 10 (step S504) so that the image brightness is within the appropriate range before proceeding to step S508. On the other hand, if the added value is within the appropriate range (step S503, Yes), the light emission quantity adjustment unit 171 directly proceeds to step S508 to allow the currently set light emission quantity of the ordinary light sources 10 to be maintained.

On the other hand, if the image is not an ordinary light image (step S501, No), the light emission quantity adjustment unit 171 adds up green (G) pixels and blue (B) pixels within a predetermined range of the special light image obtained last time (step S505). Then, the light emission quantity adjustment unit 171 determines whether the added value is within an appropriate range (step S506). If the added value is not within the appropriate range (step S506, No), the light emission quantity adjustment unit 171 makes light emission quantity adjustments of the special light sources 11 (step S507) so that the image brightness is within the appropriate range before proceeding to step S508. If the added value is within the appropriate range (step S506, Yes), the light emission quantity adjustment unit 171 directly proceeds to step S508. Then, in step S508, the light emission quantity adjustment unit 171 determines whether the light emission quantity adjustment processing has terminated and only if the processing has not terminated (step S508, No), the light emission quantity adjustment unit 171 repeats the above processing and if the processing has terminated (step S508, Yes), the light emission quantity adjustment unit 171 terminates the present processing.

In the sixth embodiment, light emission quantity adjustments are individually made for each of ordinary light images and special light images and thus, each image can be obtained as an image having individually appropriate brightness.

Light emission quantities of the ordinary light sources 10 and the special light sources 11 are adjusted in the sixth embodiment, but the present invention is not limited to this and the exposure time may be adjusted for each of ordinary light images and special light images.

Different addition operations are performed in steps S502 and S505 in the sixth embodiment described above, but the present invention is not limited to this and all pixels may be added up in each of steps S502 and S505. That is, the addition processing of steps S502 and S505 may be made common processing. In such a case, it is preferable to set each appropriate range in steps S503 and S506 differently.

Seventh embodiment

Next, as an image processing system according to the seventh embodiment of the present invention, a capsule endoscope system using a capsule endoscope as an imaging device is taken as an example. The capsule endoscope system according to the present embodiment is, like the capsule endoscope system according to any of the second to sixth embodiments, an embodiment of the image processing system according to the first embodiment described above and the concept thereof is contained in the concept of the image processing system.

In the seventh embodiment, luminance of each of ordinary light images and special light images is calculated based on each calculation formula corresponding to characteristics of output of each image as brightness information to make light emission quantity adjustments of the ordinary light sources 10 and the special light sources 11.

The light emission quantity adjustment unit 171 according to the seventh embodiment makes, like the light emission quantity adjustment unit 171 according to the sixth embodiment, light emission quantity adjustments, but performs the processing according to the light emission quantity adjustment processing procedure shown in FIG. 17. That is, the light emission quantity adjustment unit 171 determines whether the currently captured image is an ordinary light image based on control content of the observation mode controller 172 (step S601). If the image is an ordinary light image (step S601, Yes), the light emission quantity adjustment unit 171 calculates average luminance YW of all pixels (R, G, B) within a predetermined range of the ordinary light image obtained last time (step S602) according to Formula (1) below:

$$YW = 0.30 \times R + 0.59 \times G + 0.11 \times B \quad (1)$$

Then, the light emission quantity adjustment unit 171 determines whether the average luminance YW is within an appropriate range, that is, the image has appropriate brightness (step S603). If the average luminance YW is not within the appropriate range (step S603, No), the light emission quantity adjustment unit 171 makes light emission quantity adjustments of the ordinary light sources 10 (step S604) so that the image brightness is within the appropriate range before proceeding to step S608. On the other hand, if the average luminance YW is within the appropriate range (step S603, Yes), the light emission quantity adjustment unit 171 directly proceeds to step S608 to allow the currently set light emission quantity of the ordinary light sources 10 to be maintained.

On the other hand, if the image is not an ordinary light image (step S601, No), the light emission quantity adjustment unit 171 calculates average luminance based on values of green (G) pixels and blue (B) pixels within a predetermined range of the special light image obtained last time (step S605) according to Formula (2) below:

$$YN = 0.30 \times G + 0.70 \times B \quad (2)$$

Formula (2) is a formula applied when red (R) pixels are output as green (G) pixels and blue (B) pixels as blue (B) pixels.

Then, the light emission quantity adjustment unit 171 determines whether the average luminance YN is within an appropriate range (step S606). If the average luminance YN is not within the appropriate range (step S606, No), the light emission quantity adjustment unit 171 makes light emission quantity adjustments of the special light sources 11 (step S607) so that the image brightness is within the appropriate range before proceeding to step S608. If the image brightness is within the appropriate range (step S606, Yes), the light emission quantity adjustment unit 171 directly proceeds to step S608. Then, in step S608, the light emission quantity adjustment unit 171 determines whether the light emission quantity adjustment processing has terminated and only if the processing has not terminated (step S608, No), the light emission quantity adjustment unit 171 repeats the above processing and if the processing has terminated (step S608, Yes), the light emission quantity adjustment unit 171 terminates the present processing. The appropriate range in step S603 and that in step S606 may be the same or different.

In the seventh embodiment, average luminance is individually calculated using average luminance calculation formulas that are different for each of ordinary light images and special light images and light emission quantity adjustments are made based on the average luminance and thus, each image can be obtained as an image having individually appropriate brightness.

Eighth embodiment

Next, as an image processing system according to the eighth embodiment of the present invention, a capsule endoscope system using a capsule endoscope as an imaging device is taken as an example. The capsule endoscope system according to the present embodiment is, like the capsule endoscope system according to any of the second to seventh embodiments, an embodiment of the image processing system according to the first embodiment described above and the concept thereof is contained in the concept of the image processing system.

In the eighth embodiment, brightness adjustments of each piece of image data are made by performing amplification processing of pixel data corresponding to each of received ordinary light images and special light images.

FIG. 18 is a block diagram showing the configuration related to image processing of the receiving device 3 according to the eighth embodiment of the present invention. As shown in FIG. 18, the receiving device 3 includes a preprocessing circuit 203 that outputs data of each color of RGB by performing preprocessing on data D obtained by converting a radio signal transmitted from the capsule endoscope 2 by radio into a base-band signal, an image determination unit 204 that determines whether an image processed by the preprocessing unit 203 is an ordinary light image or special light image, an average luminance calculation unit 205 that calculates average luminance of a predetermined range of an image based on a determination result of the image determination unit 204, an amplification unit 206 that amplifies or attenuates each piece of image data based on a calculation result of the average luminance calculation unit 205, and a signal processing unit 207 that outputs an image processed by the amplification unit 206 after performing predetermined signal processing on the image. The receiving device 3 also includes a control unit 200 that controls the preprocessing unit 203, the image determination unit 204, the average luminance calculation unit 205, the amplification unit 206, and the signal processing unit 207. Further, the control unit 200 includes a brightness adjustment unit 201 and the brightness adjustment unit 201 makes image brightness adjustments by controlling amplification processing by the amplification unit 206 based on processing results,of the image determination unit 204 and the average luminance calculation unit 205.

The brightness adjustment processing procedure will be described with reference to the flow chart shown in FIG. 19. First, the brightness adjustment unit 201 determines whether the input image is an ordinary light image based on a determination result of the image determination unit 204 (step S701). If the image is not an ordinary light image (step S701, No), the brightness adjustment unit 201 causes the average luminance calculation unit 205 to calculate average luminance of all pixels within a predetermined range of the special light image (step 5702).

Then, the brightness adjustment unit 201 determines whether the calculated average luminance is within an appropriate range (step S703). If the average luminance is not within the appropriate range (step S703, No), the brightness adjustment unit 201 changes the amplification factor of image data by the amplification unit 206 so that the brightness of the special light image is within the appropriate range and outputs a special light image composed of image data having appropriate brightness to the signal processing unit 207 (step S704) before proceeding to step S705.

On the other hand, if the average luminance is within the appropriate range (step S703, Yes), the brightness adjustment unit 201 directly outputs each piece of pixel data to the signal processing unit 207 without amplifying the pixel data before proceeding to step S705. If, in step S701, the image is an ordinary light image (step S701, Yes), the brightness adjustment unit 201 directly proceeds to step S705. Then, in step S705, the brightness adjustment unit 201 determines whether the brightness adjustment processing has terminated and only if the processing has not terminated (step S705, No), the brightness adjustment unit 201 repeats the above processing and if the processing has terminated (step S705, Yes), the brightness adjustment unit 201 terminates the present processing.

In the eight embodiment, amplification processing of pixel data corresponding to the type of an acquired image, that is, corresponding to each of ordinary light images and special light images and thus, an image having appropriate brightness can be obtained.

The brightness adjustment unit 201 may further amplify pixel data by the signal processing unit 207 based on a calculation result of average luminance. The amplification unit 206 may perform, in addition to amplification, attenuation processing.

Further, in the eighth embodiment described above, the processing is described as processing to be performed inside the receiving device 3, but the present invention is not limited to this and amplification processing similar to that performed inside the receiving device 3 may be performed by the image display device 4. Naturally, amplification processing may be performed by the capsule endoscope 2.

The second to eighth embodiments described above have each been described by taking the capsule endoscope 2 as an example. After being inserted into a subject, the capsule endoscope 2 needs to exercise operation control of the observation mode on its own and thus is suitable for the application of the present invention.

Ninth embodiment

Next, as an image processing system according to the ninth embodiment of the present invention, a capsule endoscope system using a capsule endoscope as an imaging device is taken as an example. The capsule endoscope system according to the present embodiment is, like the capsule endoscope system according to any of the second to eighth embodiments, an embodiment of the image processing system according to the first embodiment described above and the concept thereof is contained in the concept of the image processing system.

The capsule endoscope 2 according to the present embodiment determines the light emission time of the ordinary light sources 10 or the special light sources 11 for the next imaging based on brightness of image data obtained by the last imaging. The image data obtained by the imaging is transmitted to the receiving device 3 outside the subject 1 through a radio signal by the transmitting circuit 55 via the transmitting antenna 56. The receiving device 3 records the image data received from the capsule endoscope 2 in, for example, the portable recording medium 5. At this point, the receiving device 3 works not to store images whose brightness level is extremely low or high. Accordingly, images that are not effective in reading X-rays inside the subject 1 (images not contained within an allowable range) such as underexposed images that are dark and blurred as a whole and overexposed images that are whitened as a whole can be discarded.

Subsequently, a capsule endoscope system according to the ninth embodiment will be described in detail together with drawings. The capsule endoscope system according to the ninth embodiment is similar to that of one of the above embodiments. In the present embodiment, however, as shown in FIG. 20, the exposure time measuring unit 71 (see FIG. 7) in the system controller 54 of the capsule endoscope 2 is replaced by a brightness information detection unit 71A. The brightness information detection unit 71A detects a value (also called an amount of exposure) obtained by integrating signal strength of pixels inside a predetermined region of an image signal read from, for example, the imaging element 14 as brightness information. FIG. 20 is a block diagram showing the configuration of a capsule endoscope according to the ninth embodiment.

Next, the operation of a capsule endoscope system according to the present embodiment will be described in detail using drawings. FIG. 21 is a flow chart showing an outline operation of the capsule endoscope according to the ninth embodiment. FIG. 22 is a flow chart showing the outline operation of the receiving device 3 according to the ninth embodiment. The operation shown in FIG. 21 is repeated until the battery in the capsule endoscope 2 runs out.

As shown in FIG. 21, after being activated, the capsule endoscope 2 first selects the ordinary light observation mode (step S901) and to emit light from the ordinary light sources 10 (step S902). Subsequently, the capsule endoscope 2 drives the imaging unit 52 to acquire image data (step S903) and transmits the acquired image data to the receiving device 3 through a radio signal (step S904).

Next, the capsule endoscope 2 switches the imaging mode to the ordinary light observation mode or the special light observation mode (step S905). If, for example, the current imaging mode is the ordinary light observation mode, the observation mode is switched to the special light observation mode and if the current imaging mode is the special light observation mode, the observation mode is switched to the ordinary light observation mode. Subsequently, the capsule endoscope 2 determines whether the observation mode after the switching, that is, the observation mode for the next photographing is the special light observation mode (step S906).

If, as a result of the determination in step S906, the current observation mode is the ordinary light observation mode (step S906, No), the capsule endoscope 2 detects brightness information of the image from all components of R components, G components, and B components in the ordinary light image acquired last time (step S907). Subsequently, the capsule endoscope 2 calculates the light emission time of the ordinary light sources 10 from the detected brightness information (step S908) and causes the ordinary light sources 10 to emit light for the calculated light emission time (step S909) before returning to step S903. If the light emission time calculated in step S908 is larger than a maximum value of the light emission time preset as an upper limit, the capsule endoscope 2 causes the ordinary light sources 10 to emit light, for example, for the maximum value of the light emission time.

On the other hand, if, as a result of the determination in step S906, the current observation mode is the special light observation mode (step S906, Yes), the capsule endoscope 2 detects brightness information of the image from G components and B components in the ordinary light image or special light image acquired immediately before, that is, color components forming a special light image (step S910), calculates the light emission time of the special light sources 11 from the detected brightness information (step S911) and causes the special light sources 11 to emit light for the calculated light emission time (step S912) before returning to step S903. If the light emission time calculated in step S912 is larger than a maximum value of the light emission time preset as an upper limit, the capsule endoscope 2 causes the ordinary light sources 10 to emit light, for example, for the maximum value of the light emission time.

As shown in FIG. 22, the receiving device 3 waits to receive image data from the capsule endoscope 2 (step S921, No). When image data is received from the capsule endoscope 2 (step S921, Yes), the receiving device 3 determines whether the received image is a special light image (step S922). If the received image is not a special light image, that is, an ordinary light image (step S922, No), the receiving device 3 receives an allowable range of brightness for an ordinary light image (step S923). On the other hand, if the received image is a special light image (step S922, Yes), the receiving device 3 receives an allowable range of brightness for a special light image (step S924). The allowable range of brightness for an ordinary light image and that of brightness for a special light image can be realized by, for example, presetting the upper limit and lower limit of each range. The upper limit and lower limit of each range are stored in, for example, a memory (not shown) in the receiving device 3 in advance.

Next, the receiving device 3 derives brightness information of an image from a pixel value of pixels contained in a predetermined region of the target image (step S925) and determines whether the brightness of the image identified from the brightness information is included in the allowable range identified in step S923 or S924 (step S926). If, as a result of the determination in step S926, the brightness of the target image is included in the allowable range (step S926, Yes), the receiving device 3 performs image processing such as synchronization processing and compression processing on the target image (step S927) and stores image data after the image processing in the recording medium 5 (step S928). On the other hand, if the brightness of the target image is not included in the allowable range (step S926, No), the receiving device 3 discards the target image data (step S929).

Then, the receiving device 3 determines whether any termination instruction of the operation has been input from, for example, a user (step S930) and if the termination instruction has been input (step S930, Yes), the receiving device 3 terminates the operation shown in FIG. 22. On the other hand, if no termination instruction has been input (step S930, No), the receiving device 3 returns to step S921 to perform the operation that follows.

In the present embodiment, as described above, appropriate control not to store image data that does not have appropriate brightness can be performed by the receiving device 3 in a stable fashion based on brightness of the image. As a result, various kinds of processing on image data that is not effective in reading X-rays and a region where image data not effective in reading X-rays is stored can be eliminated so that processing can be slimmed and the storage region can be used more effectively.

Tenth embodiment

Next, as an image processing system according to the tenth embodiment of the present invention, a capsule endoscope system using a capsule endoscope as an imaging device is taken as an example. The capsule endoscope system according to the present embodiment is, like the capsule endoscope system according to any of the second to ninth embodiments, an embodiment of the image processing system according to the first embodiment described above and the concept thereof is contained in the concept of the image processing system.

The capsule endoscope 2 according to the present embodiment determines the light emission time of the ordinary light sources 10 or the special light sources 11 for the next imaging based on brightness of image data obtained by the last imaging. The image data obtained by the imaging is transmitted to the receiving device 3 outside the subject 1 through a radio signal by the transmitting circuit 55 via the transmitting antenna 56 and stored in predetermined storage (for example, the recording medium 5). The stored image data is loaded into the image display device 4 via a communication interface (such as USB and LAN) connecting a cradle and the image display device 4 when, for example, the receiving device 3 is connected to the cradle (not shown). The image display device 4 performs image processing functions such as the motion detection function that detects image motion (or movement of the capsule endoscope 2 predicted based on image changes) and the red detection function that determines whether there is any red portion in an image or detects a region of a red portion in an image on the input image data.

The motion detection function calculates a scalar quantity (an absolute value) of a motion vector between consecutive images and if the quantity is larger than a preset threshold, selects the target image as a display target, that is, an image for reading X-rays. Images excluded from display targets are stocked, for example, in a predetermined storage region while maintaining chronological information of consecutive images.

Cases when a large scalar quantity is calculated include, for example, a case when an imaging window of the capsule endoscope 2 is directed toward the direction of emptiness from a state in which the imaging window is close to in-vivo tissues (hereinafter, referred to as a first case) and a case when an observation window comes into contact with in-vivo tissues from a state in which the imaging window is in the direction of emptiness (hereinafter, referred to as a second case). In a state in which the imaging window is close to in-vivo tissues, an object (in-vivo tissues) can be clearly imaged with a small illuminating light quantity. Thus, in the first case, one or several images captured immediately after the observation window is directed toward the direction of emptiness will be underexposed dark images. While such dark images are not appropriate for reading X-rays, the scalar quantity thereof becomes a large value because such dark images have a large motion vector with regard to images captured immediately before when the observation window is close to in-vivo tissues. As a result, such dark images will be selected as display target images. On the other hand, the distance between the imaging unit and an object is long in a state in which the imaging window is in the direction of emptiness and thus, a bright image cannot be obtained unless illuminated with a large illuminating light quantity. Thus, in the second case, one or several images captured immediately after the observation window being close to in-vivo tissues will be overexposed too bright images. While such too bright images are not appropriate for reading X-rays, the scalar quantity thereof becomes a large value because such too bright images have a large motion vector with regard to images captured immediately before when the observation window is in the direction of emptiness. As a result, such too bright images will be selected as display target images.

Thus, in the present embodiment, whether to select a target image as a display target is determined based on, in addition to the scalar quantity of a motion vector between consecutive images, brightness information of each image. Accordingly, dark images or too bright images that are not appropriate for reading X-rays can be prevented from being selected as display targets.

For the red detection function, malfunctioning of the algorithm thereof may be triggered by an image whose brightness is lacking or excessive. This is because the white balance of an image changes depending on the level of contrast such as the R component (red component) being dominant over other components (G and B components) in a dark image. That is, if the white balance of an image is disturbed, the red detection function that detects reddish images (images containing many red regions or images strong in the R component) by an algorithm based on the relative value of each color component may evaluate the image whose white balance is disturbed differently from colors in real space. As a result, even if red is strong in real space, an image capturing the redness may be evaluated as an image strong in red or even if red is not strong in real space, an image capturing the redness may be evaluated as an image strong in red.

Thus, the present embodiment is configured to perform red detection only for images having a certain level of uniform brightness. Accordingly, execution of red detection of an image whose white balance is significantly disturbed can be avoided so that the operation of the red detection function can be stabilized.

The operation of a capsule endoscope system according to the present embodiment will be described below in detail using drawings. FIG. 23 is a flow chart showing the outline operation of the image display device 4 according to the tenth embodiment. FIG. 24 is a flow chart showing the outline operation of an example of the image processing function (motion detection function) executed by the image display device in the tenth embodiment. FIG. 25 is a flow chart showing the outline operation of another example of the image processing function (red detection function) executed by the image display device in the tenth embodiment.

First, as shown in FIG. 23, the image display device 4 waits for input of image data from the receiving device 3 via a cradle (step S1001, No) and when image data is input (step S1001, Yes), executes the image processing function for the image data (step S1002). Image data input in step S1001 is not limited to one piece of image data and may be a group of image data arranged chronologically. The image processing function executed in step S1002 includes, for example, the motion detection function and the red detection function.

Next, the image display device 4 causes the user to read intra-subject images by performing image display processing to display the image processed by using the image processing function (step S1003). Then, the image display device 4 determines whether any termination instruction of the operation has been input from, for example, a user (step S1004) and if the termination instruction has been input (step S1004, Yes), the image display device 4 terminates the operation. On the other hand, if no termination instruction has been input (step S1004, No), the image display device 4 returns to step S1001 to perform the operation that follows. However, the step to which the image display device 4 returns is not limited to step S1001 and may be step S1002 or S1003.

Next, the motion detection function will be described as an example of the image processing function executed in step S1002 in FIG. 23. When the motion detection function is executed, as shown in FIG. 24, the image display device 4 selects one piece of input image data (step S1011) and detects brightness information of the image (step S1012). If, for example, image data is arranged chronologically, image data is selected in chronological order.

Next, the image display device 4 determines whether the brightness of the target image is within a preset allowable range based on the detected image brightness information (step S1013) and if the brightness of the target image is not within the allowable range (step S1013, No), sets the target image data as image data excluded from display targets (step S1017) before proceeding to step S1018.

On the other hand, if the brightness of the target image is within the allowable range (step S1013, Yes), the image display device 4 calculates a motion vector between the target image data and the image data chronologically immediately before (step S1014). Subsequently, the image display device 4 determines whether the scalar quantity (absolute value) of the calculated motion vector is equal to a preset threshold or more (step S1015) and if the scalar quantity is not equal to the preset threshold or more (step S1015, No), sets the target image data as image data excluded from display targets (step S1017) before proceeding to step S1018.

On the other hand, if the scalar quantity (absolute value) of the calculated motion vector is equal to the threshold or more (step S1015, Yes), the image display device 4 selects the target image as a display target image (step S1016). The selection of a display target image can be realized by, for example, attaching a flag indicating a display target to image data or recording an image to be displayed in a recording region such as another folder.

Then, the image display device 4 determines whether the above processing has been performed on all input image data (step S1018) and if the above processing has been performed on all input image data (step S1018, Yes), returns to the operation shown in FIG. 23. On the other hand, if there is image data that is not yet processed (step S1018, No), the image display device 4 returns to step S1011 and performs the operation that follows.

Next, the red detection function will be described as an example of the image processing function executed in step S1002 in FIG. 23. When the red detection function is executed, as shown in FIG. 25, the image display device 4 selects one piece of input image data (step S1021) and detects brightness information of the image (step S1022). If, for example, image data is arranged chronologically, image data is selected in chronological order.

Next, the image display device 4 determines whether the brightness of the target image is within a preset allowable range based on the detected image brightness information (step S1023) and if the brightness of the target image is not within the allowable range (step S1023, No), sets the target image data as image data excluded from red detection targets (step S1027) before proceeding to step 1028.

On the other hand, if the brightness of the target image is within the allowable range (step S1023, Yes), the image display device 4 identifies the threshold of a color evaluation function in accordance with brightness information managed in a memory (not shown) or the like in advance (step S1024) and performs red detection of the target image using the threshold (step S1025). The image display device 4 stores a detected result in the same time sequence as that of the image data (step S1026).

Then, the image display device 4 determines whether the above processing has been performed on all input image data (step S1028) and if there is image data that is not yet processed (step S1028, No), the image display device 4 returns to step S1021 and performs the operation that follows. On the other hand, if the processing has been performed on all image data (step S1028, Yes), the image display device 4 generate red bar images from red detection results stored in the time sequence in step S1026 (step S1029) and then, returns to the operation shown in FIG. 23. Red bars are bar-shaped images enabling recognition of red detection results of images in a time sequence.

According to the present embodiment, as described above, appropriate control in accordance with image brightness is enabled by the image processing function being operated based on image brightness so that image processing can be performed on image data in a stable fashion.

In the tenth embodiment, the image display device 4 is configured to control the operation based on whether the value of brightness information is within a range (allowable range) of the preset upper limit and lower limit, but the present invention is not limited to this and various modifications can be made. For example, the amount of change of the value of image brightness information between consecutive images may be calculated to configure the image display device 4 to operate in accordance with the amount of change. In this case, for example, an image whose amount of change from the previous image is larger than a preset threshold may be selected as a display target image or a red detection target image.

Also in the tenth embodiment, the image display device 4 is configured to perform red detection by selecting images whose value of brightness information is included in an allowable range as targets for red detection, but the present invention is not limited to this and various modifications can be made. For example, the image display device 4 may be configured so that the red detection function changes the threshold of a color evaluation coefficient used for red detection in accordance with the value of brightness information. Accordingly, the operating precision of the red detection function can further be improved. Correspondences between the threshold of the color evaluation function and brightness information may be derived in advance and managed in a table in a memory.

## Eleventh embodiment

Next, as an image processing system according to the eleventh embodiment of the present invention, a capsule endoscope system using a capsule endoscope as an imaging device is taken as an example. The capsule endoscope system according to the present embodiment is, like the capsule endoscope system according to any of the second to tenth embodiments, an embodiment of the image processing system according to the first embodiment described above and the concept thereof is contained in the concept of the image processing system.

In the capsule endoscope system according to the present embodiment, for example, the capsule endoscope 2 acquires ordinary light images. An image obtained by the capsule endoscope 2 is input into the image display device 4 via the receiving device 3. The image display device 4 generates a special light image by using G components and B components from the input ordinary light image. The image display device 4 also performs predetermined image processing on the ordinary light image and special light image and presents a result of the processing and the images to the user.

If image data captured in ordinary light observation mode by using the ordinary light sources 10 contains many R components, G and B components may be insufficient. In such a case, while brightness of an ordinary light image is sufficient, brightness of a special light image generated from the ordinary light image is at a low level. Thus, in the present embodiment, the illuminating unit 51B is controlled so that G and B components in an image obtained by the next imaging are sufficient for generation of a special light image based on brightness of the image obtained by the last imaging. Accordingly, an ordinary light image and a special light image can be obtained from an image obtained in one imaging.

A capsule endoscope system according to the present embodiment will be described below in detail using drawings. The capsule endoscope system according to the present embodiment is like that of one of the above embodiments. However, as shown in FIG. 26, the special light sources 11 in the illuminating unit 51 of the capsule endoscope 2 are omitted in the present embodiment. Moreover, the system controller 54B is replaced by a system controller 54B including a brightness information detection unit 73 that detects brightness information of an image and a flag attachment unit 74 that attaches a flag (an ordinary light image flag or special light image flag) to the image based on the detected brightness information. FIG. 26 is a block diagram showing the configuration of the capsule endoscope according to the eleventh embodiment.

Next, the operation of a capsule endoscope system according to the present embodiment will be described in detail using drawings. FIG. 27 is a flow chart showing the outline operation of a capsule endoscope according to the eleventh embodiment. FIG. 28 is a flow chart showing the outline operation of a receiving device according to the eleventh embodiment. FIG. 29 is a flow chart showing the outline operation of an image display device according to the eleventh embodiment. The operation shown in FIG. 27 is repeated until the battery in the capsule endoscope 2 runs out.

As shown in FIG. 27, after being activated, the capsule endoscope 2 first emits light from the ordinary light sources 10 (step S1101) and subsequently drives the imaging unit 52 to acquire image data (step S1102). Next, the capsule endoscope 2 detects brightness information of an ordinary light image (hereinafter, referred to as ordinary light image brightness information) from R, G, and B components of the acquired image data (step S1103) and then detects brightness information of a special light image (hereinafter, referred to as special light image brightness information) composed of G and B components of the image data (step S1104).

Next, the capsule endoscope 2 determines whether the value of the ordinary light image brightness information detected in step S1103 is within a preset allowable range (step S1105) and if the value is within the allowable range (step S1105, Yes), attaches an ordinary light image flag indicating that the image data is an ordinary light image effective in reading X-rays to the image data (step S1106). On the other hand, if the value of the ordinary light image brightness information is not within the allowable range (step S1105, No), the capsule endoscope 2 directly proceeds to step S1107.

Next, the capsule endoscope 2 determines whether the value of the special light image brightness information detected in step S1104 is within a preset allowable range (step S1107) and if the value is within the allowable range (step S1107, Yes), attaches a special light image flag indicating that the image data is image data from which a special light image can be generated to the image data (step S1108). On the other hand, if the value of the special light image brightness information is not within the allowable range (step S1107, No), the capsule endoscope 2 directly proceeds to step S1109. Instead of the ordinary light image flag and special light image generation flag described above, calculated ordinary light image brightness information and/or special light image brightness information may be attached to image data.

Next, the capsule endoscope 2 transmits the image data to the receiving device 3 (step S1109). Subsequently, the capsule endoscope 2 calculates the light emission time of the ordinary light sources 10 for the next imaging from the special light image brightness information (step S1110) and emits light from the ordinary light sources 10 for the calculated light emission time (step S1111). Then, the capsule endoscope 2 returns to step S1102 and hereafter performs the same operation. If the light emission time calculated in step S1110 is larger than a maximum value of the light emission time preset as an upper limit, the capsule endoscope 2 causes the ordinary light sources 10 to emit light, for example, for the maximum value of the light emission time.

As shown in FIG. 28, the receiving device 3 waits to receive image data from the capsule endoscope 2 (step S1121, No). When image data is received from the capsule endoscope 2 (step S1121, Yes), the receiving device 3 determines whether at least one of the ordinary light image flag and special light image flag is attached to the received image data (step S1122) and if no flag is attached (step S1122, No), discards the image data without storing the image data (step S1125).

On the other hand, if the special light image generation flag is attached to the image data (step S1122, Yes), the receiving device 3 performs predetermined image processing such as synchronization processing and compression processing on the image data (step S1123) and stores the image data after the image processing in the recording medium 5 (step S1124).

Then, the receiving device 3 determines whether any termination instruction of the operation has been input from, for example, a user (step S1126) and if the termination instruction has been input (step S1126, Yes), the receiving device 3 terminates the operation shown in FIG. 28. On the other hand, if no termination instruction has been input (step S1126, No), the receiving device 3 returns to step S1121 to perform the operation that follows.

As shown in FIG. 29, the image display device 4 waits for input of image data from the receiving device 3 via a cradle (step S1131, No) and when image data is input (step S1131, Yes), selects one piece of input image data (step S1132) and determines whether a special light image flag is attached to the image data (step S1133). If, as a result of the determination of step S1133, no special light image flag is attached to the image data (step S1133, No), the image display device 4 directly proceeds to step S1135. On the other hand, if a special light image flag is attached to the image data (step S1133, Yes), the image display device 4 generates a special light image from G and B components of the image data (step S1134) before proceeding to step S1135.

In step S1135, the image display device 4 stores the image data. Thus, if a special light image is generated in step S1134, in addition to an ordinary light image, the image display device 4 stores the ordinary light image and special light image in step S1135.

Next, the image display device 4 determines whether the above processing has been performed on all input image data (step S1136) and if there is image data that is not yet processed (step S1136, No), the image display device 4 returns to step S1132 and performs the operation that follows. On the other hand, if the processing has been performed on all image data (step S1136, Yes), the image display device 4 determines whether any termination instruction of the operation has been input from, for example, a user (step S1137) and if the termination instruction has been input (step S1137, Yes), the image display device 4 terminates the operation. On the other hand, if no termination instruction has been input (step S1137, No), the image display device 4 returns to step S1131 to perform the operation that follows.

In the present embodiment, as described above, not only the capsule endoscope 2, but also the receiving device 3 and the image display device 4 can operate on the basis of information based on brightness (such as a flag and brightness information) attached to image data by the capsule endoscope 2 and thus, image data itself can be generated and processing on the generated image data can be performed in a stable fashion.

Brightness of images obtained by the imaging unit 52 is adjusted by controlling the exposure time of the imaging unit 52 in accordance with brightness of images in the second to eighth embodiments described above and brightness of images obtained by the imaging unit 52 is adjusted by controlling the illumination time of the illuminating unit 51 in accordance with brightness of images in the ninth to eleventh embodiment described above. However, the present invention is not limited to such examples and it is easy for those skilled in the art to partially recombine configurations among the above embodiments such as adjusting brightness of images obtained by the imaging unit 52 by controlling the illumination time of the illuminating unit 51 in accordance with brightness of images in the second to eighth embodiments and adjusting brightness of images obtained by the imaging unit 52 by controlling the exposure time of the imaging unit 52 in accordance with brightness of images in the ninth to eleventh embodiments and thus, a detailed description thereof is omitted here.

Reference Signs List

| | |
|---|---|
| 100 | IMAGE PROCESSING SYSTEM |
| 101 | IMAGE GENERATION UNIT |
| 102 | DISPLAY UNIT |
| 103 | ILLUMINATING UNIT |
| 104 | IMAGING UNIT |
| 105 | IMAGE PROCESSING UNIT |
| 106 | BRIGHTNESS DETECTION UNIT |
| 107 | CONTROL UNIT |
| 1 | SUBJECT |
| 2 | CAPSULE ENDOSCOPE |
| 3 | RECEIVING DEVICE |
| 3a to 3h | RECEIVING ANTENNA |
| 4 | IMAGE DISPLAY DEVICE |
| 10, 110 | ORDINARY LIGHT SOURCE |
| 11 | SPECIAL LLIGHT SOURCE |
| 12 | TRANSPARENT FIXING MEMBER |
| 13 | OPTICAL LENS |
| 14 | IMAGING ELEMENT |
| 20 | DOME-SHAPED TRANSPARENT COVER |
| 21 | CAPSULE CASING |
| 23 | SUBSTRATE |
| 40 | SURFACE LAYER OF MUCOSA |
| 41 | DEEP PART OF MUCOSA |
| 42 | INTERNAL TISSUE |
| 43 | CAPILLARY |
| 44 | BLOOD VESSEL |
| 51 | ILLUMINATING UNIT |
| 52 | IMAGING UNIT |
| 53 | STATE DETECTION UNIT |
| 54, 54B | SYSTEM CONTROLLER |
| 55 | TRANSMITTING CIRCUIT |
| 56 | TRANSMITTING ANTENNA |
| 57 | POWER SUPPLY CIRCUIT |
| 61 | LIGHT SOURCE CONTROL CIRCUIT |
| 62 | IMAGING ELEMENT CONTROL CIRCUIT |
| 63 | SENSOR UNIT |
| 64 | SENSOR UNIT CONTROL CIRCUIT |
| 71 | EXPOSURE TIME MEASURING UNIT |

| | |
|---|---|
| 72, 172 | OBSERVATION MODE CONTROLLER |
| 73 | BRIGHTNESS INFORMATION DETECTION UNIT |
| 74 | FLAG ATTACHMENT UNIT |
| 111 | WIDE-DIRECTIVITY SPECIAL LIGHT SOURCE |
| 112 | NARROW-DIRECTIVITY SPECIAL LIGHT SOURCE |
| 171 | LIGHT EMISSION QUANTITY ADJUSTMENT UNIT |
| 201 | BRIGHTNESS ADJUSTMENT UNIT |
| 203 | PREPROCESSING UNIT |
| 204 | IMAGE DETERMINATION UNIT |
| 205 | AVERAGE LUMINANCE CALCULATION UNIT |
| 206 | AMPLIFICATION UNIT |
| 207 | SIGNAL PROCESSING UNIT |

## Claims

**1.** A capsule endoscope system (2), comprising:

an illuminating unit (51) including an ordinary light source (10) for illuminating an object by ordinary light of a preset quantity (S101) as an ordinary light observation mode and a special light source (11) for illuminating the object by special light of a preset quantity (S106) as a special light observation mode;
an imaging unit (52) configured to capture an image of the object in each observation mode by receiving reflected light from the object; and
a system controller (54) including an exposure time measuring unit (71) configured to measure an exposure time when the imaging unit (52) captures an image in the ordinary light observation mode as brightness information, and an observation mode controller (72) configured to control operations of the ordinary light observation mode for capturing an ordinary light image and the special light observation mode for capturing a special light image, based on the exposure time measured by the exposure time measuring unit (71),
**characterized in that**
the observation mode controller (72) is configured to alternately switch between the ordinary light observation mode and the special light observation mode (S101, S106, S102), and to determine whether the next observation mode is the special light observation mode (S104), if not continue to the ordinary light observation mode, if yes determine whether the exposure time ($\Delta$T2) of the two previous ordinary light observation modes are both successively equal to or more than a specified value ($\Delta$T3), wherein the specified value is smaller than a maximum exposure time setting value (tmax), if no perform the special light observation mode (S106), if yes perform the ordinary light observation mode instead of the special light observation mode.

**2.** The capsule endoscope system (2) according to claim 1, wherein
the special light source (11) includes a narrow-directivity light source (112) having narrow directivity with respect to an optical axis direction of the imaging unit and a wide-directivity light source (111) having wide directivity, and
if the exposure time in the ordinary light observation mode exceeds a predetermined value successively a plurality of times till a last time, the observation mode controller (72) is configured to cause the next imaging operation to perform in the special light observation mode by causing only the wide-directivity light sources to emit light and if the predetermined value is not exceeded the plurality of times, the observation mode controller (72) is configured to cause the next imaging operation to perform in the special light observation mode by causing the wide-directivity light source and the narrow-directivity light source to emit light.

**3.** The capsule endoscope system (2) according to claim 2, further comprising a state detection unit (53) configured to detect whether at least the imaging unit (52) and the illuminating unit (51) are in a liquid, wherein
if the exposure time in the ordinary light observation mode exceeds the predetermined value successively the plurality of times till the last time and the state detection unit detects that the imaging unit and the illuminating unit are in the liquid, the observation mode controller (72) is configured to cause the next imaging operation to perform in the special light observation mode by causing only the wide-directivity light source to emit light and if the exposure time in the ordinary light observation mode exceeds the predetermined value successively the plurality of times till the last time and the state detection unit detects that the imaging unit and the illuminating unit is not in the liquid, the observation mode controller (72) is configured to stop the next imaging operation in the special light observation mode to cause the imaging operation to perform in the ordinary light observation mode in place of the special light observation mode.

4. The capsule endoscope system (2) according to claim 1, wherein
the ordinary light source (10) is a white light source and
the special light source (11) is a narrow-band light source that emits spectral light of a specific color component.

5. The capsule endoscope system (2) according to claim 4, wherein
the narrow-band light source is configured to emit the spectral light having blue and green as the specific color component.

6. The capsule endoscope system (2) according to claim 1, further comprising:

a transmission unit (55) configured to transmit the images captured by the imaging unit (52).

**Patentansprüche**

1. Kapselendoskopsystem (2), das umfasst:

eine Beleuchtungseinheit (51), die eine Normallichtquelle (10) zum Beleuchten eines Objekts mit Normallicht einer vorbestimmten Menge (S101) als einen Normallichtbeobachtungsmodus und eine Speziallichtquelle (11) zum Beleuchten des Objekts mit Speziallicht einer vorbestimmten Menge (S106) als einen Speziallichtbeobachtungsmodus umfasst;
eine Bildgebungseinheit (52), die dazu eingerichtet ist, durch den Empfang von von dem Objekt reflektiertem Licht ein Bild des Objekts in jedem Beobachtungsmodus aufzunehmen; und
eine Systemsteuerung (54) mit einer Belichtungszeitmesseinheit (71), die dazu eingerichtet ist, eine Belichtungszeit als eine Helligkeitsinformation zu messen, wenn die Bildgebungseinheit (52) ein Bild in dem Normallichtbeobachtungsmodus aufnimmt, und eine Beobachtungsmodussteuerung (72), die dazu eingerichtet ist, Operationen des Normallichtbeobachtungsmodus zum Aufnehmen eines Bilds bei Normallicht und des Speziallichtbeobachtungsmodus zum Aufnehmen eines Bilds bei Speziallicht auf der Basis der von der Belichtungszeitmesseinheit (71) gemessenen Belichtungszeit zu steuern,
**dadurch gekennzeichnet, dass**
die Beobachtungsmodussteuerung (72) dazu eingerichtet ist, abwechselnd zwischen dem Normallichtbeobachtungsmodus und dem Speziallichtbeobachtungsmodus umzuschalten (S101, S106, S102) und zu bestimmen, ob der nächste Beobachtungsmodus der Speziallichtbeobachtungsmodus ist (S104), falls nicht, mit dem Normallichtbeobachtungsmodus fortzufahren, falls ja, zu bestimmen, ob die Belichtungszeiten ($\Delta$T2) der zwei vorhergehenden Normallichtbeobachtungsmodi beide aufeinanderfolgend gleich einem oder mehr als ein spezifizierter Wert ($\Delta$T3) sind, wobei der spezifizierte Wert kleiner als ein maximaler Belichtungszeiteinstellwert (tmax) ist, falls nicht, den Speziallichtbeobachtungsmodus durchzuführen (S106), falls ja, den Normallichtbeobachtungsmodus anstelle des Speziallichtbeobachtungsmodus durchzuführen.

2. Kapselendoskopsystem (2) nach Anspruch 1, bei dem
die Speziallichtquelle (11) eine Lichtquelle (112) mit einer engen Richtcharakteristik, die bezüglich einer Richtung einer optischen Achse der Bildgebungseinheit eine enge Richtcharakteristik hat, und eine Lichtquelle (111) mit einer breiten Richtcharakteristik, die eine breite Richtcharakteristik hat, umfasst, und
falls die Belichtungszeit in dem Normallichtbeobachtungsmodus aufeinanderfolgend mehrfach bis zu einem letzten Mal einen vorbestimmten Wert überschreitet, die Beobachtungsmodussteuerung (72) dazu eingerichtet ist, zu veranlassen, dass die nächste Bildgebungsoperation in dem Speziallichtbeobachtungsmodus durchgeführt wird, indem nur die Lichtquelle mit einer breiten Richtcharakteristik zur Emission von Licht veranlasst wird, und, falls der vorbestimmte Wert nicht mehrfach überschritten wird, die Beobachtungsmodussteuerung (72) dazu eingerichtet ist, zu veranlassen, dass die nächste Bildgebungsoperation in dem Speziallichtbeobachtungsmodus durchgeführt wird, indem die Lichtquelle mit einer breiten Richtcharakteristik und die Lichtquelle mit einer engen Richtcharakteristik zur Emission von Licht veranlasst werden.

3. Kapselendoskopsystem (2) nach Anspruch 2, das ferner eine Zustandserfassungseinheit (53) umfasst, die dazu eingerichtet ist, zu erfassen, ob sich zumindest die Bildgebungseinheit (52) und die Beleuchtungseinheit (51) in einer Flüssigkeit befinden, wobei
falls die Belichtungszeit in dem Normallichtbeobachtungsmodus aufeinanderfolgend mehrfach bis zum letzten Mal den vorbestimmten Wert überschreitet und die Zustandserfassungseinheit erfasst, dass sich die Bildgebungseinheit und die Beleuchtungseinheit in der Flüssigkeit befinden, die Beobachtungsmodussteuerung (72) dazu eingerichtet ist, zu veranlassen, dass die nächste Bildgebungsoperation in dem Speziallichtbeobachtungsmodus durchgeführt wird, indem nur die Lichtquelle mit einer breiten Richtcharakteristik zur Emission von Licht veranlasst wird, und, falls die Belichtungszeit in dem Normallichtbeobachtungsmodus aufeinanderfolgend mehrfach bis zum letzten Mal den vorbestimmten Wert überschreitet und die Zustandserfassungseinheit erfasst, dass sich die Bildgebungseinheit und die Beleuchtungseinheit nicht in der Flüssigkeit befinden, die Beobachtungsmodussteuerung (72) dazu eingerichtet ist, die nächste Bildgebungsoperation in dem Speziallichtbeobachtungsmodus zu stoppen, um zu veranlassen, dass die Bildgebungsoperation in dem Normallichtbeobachtungsmodus statt in dem Speziallichtbeobachtungsmodus durchgeführt wird.

**4.** Kapselendoskopsystem (2) nach Anspruch 1, bei dem
die Normallichtquelle (10) eine Weißlichtquelle und
die Speziallichtquelle (11) eine Schmalband-Lichtquelle ist, die Spektrallicht einer spezifischen Farbkomponente emittiert.

**5.** Kapselendoskopsystem (2) nach Anspruch 4, bei dem
die Schmalband-Lichtquelle dazu eingerichtet ist, das Spektrallicht zu emittieren, das Blau und Grün als die spezifische Farbkomponente hat.

**6.** Kapselendoskopsystem (2) nach Anspruch 1, das ferner umfasst:

eine Sendeeinheit (55), die dazu eingerichtet ist, die von der Bildgebungseinheit (52) aufgenommenen Bilder zu senden.

## Revendications

**1.** Système (2) d'endoscope de type capsule, comprenant :

une unité d'éclairage (51) contenant une source de lumière ordinaire (10) destinée à éclairer un objet avec une lumière ordinaire d'une quantité préétablie (S101) en tant que mode d'observation à la lumière ordinaire et une source de lumière spéciale (11) destinée à éclairer l'objet avec une lumière spéciale d'une quantité préétablie (S106) en tant que mode d'observation à la lumière spéciale ;
une unité d'imagerie (52) configurée pour capturer une image de l'objet dans chaque mode d'observation en recevant la lumière réfléchie de l'objet ; et
un contrôleur (54) de système incluant une unité (71) de mesure de temps d'exposition configurée pour mesurer un temps d'exposition lorsque l'unité d'imagerie (52) capture une image dans le mode d'observation à la lumière ordinaire en tant qu'information de luminosité, et un contrôleur (72) de mode d'observation configuré pour commander les fonctionnements du mode d'observation à la lumière ordinaire destiné à capturer une image à la lumière ordinaire et du mode d'observation à la lumière spéciale destiné à capturer une image à la lumière spéciale, sur la base du temps d'exposition mesuré par l'unité (71) de mesure de temps d'exposition,
**caractérisé en ce que**
le contrôleur (72) de mode d'observation est configuré pour commuter alternativement entre le mode d'observation à la lumière ordinaire et le mode d'observation à la lumière spéciale (S101, S106, S102), et pour déterminer si le mode d'observation suivant est le mode d'observation à la lumière spéciale (S104), si non continuer le mode d'observation à la lumière ordinaire, si oui déterminer si les temps d'exposition ($\Delta T2$) des deux modes d'observation à la lumière ordinaire précédents sont tous deux successivement égaux ou supérieurs à une valeur spécifiée ($\Delta T3$), dans lequel la valeur spécifiée est inférieure à une valeur d'établissement de temps d'exposition maximal (tmax), si non réaliser le mode d'observation à la lumière spéciale (S106), si oui réaliser le mode d'observation à la lumière ordinaire au lieu du mode d'observation à la lumière spéciale.

**2.** Système (2) d'endoscope de type capsule selon la revendication 1, dans lequel
la source de lumière spéciale (11) comprend une source de lumière (112) à directivité étroite présentant une directivité étroite par rapport à une direction d'axe optique de l'unité d'imagerie et une source de lumière (111) à directivité large présentant une directivité large, et
si le temps d'exposition dans le mode d'observation à la lumière ordinaire dépasse une valeur prédéterminée successivement une pluralité de fois jusqu'à la dernière fois, le contrôleur (72) de mode d'observation est configuré pour amener l'opération d'imagerie suivante à s'exécuter dans le mode d'observation à la lumière spéciale en amenant uniquement les sources de lumière à directivité large à émettre une lumière et si la valeur prédéterminée n'est pas dépassée la pluralité de fois, le contrôleur (72) de mode d'observation est configuré pour amener l'opération d'imagerie suivante à s'exécuter dans le mode d'observation à la lumière spéciale en amenant la source de lumière à directivité large et la source de lumière à directivité étroite à émettre de la lumière.

**3.** Système (2) d'endoscope de type capsule selon la revendication 2, comprenant en outre une unité (53) de détection d'état configurée pour détecter si au moins l'unité d'imagerie (52) et l'unité d'éclairage (51) se trouvent dans un liquide, dans lequel
si le temps d'exposition dans le mode d'observation à la lumière ordinaire dépasse la valeur prédéterminée successivement la pluralité de fois jusqu'à la dernière fois et que l'unité de détection d'état détecte que l'unité d'imagerie et l'unité d'éclairage se trouvent dans le liquide, le contrôleur (72) de mode d'observation est configuré pour amener la prochaine opération d'imagerie à s'exécuter dans le mode d'observation à la lumière spéciale en amenant uniquement la source de lumière à directivité large à émettre de la lumière et si le temps d'exposition dans le mode d'observation à la lumière ordinaire dépasse la valeur prédéterminée successivement la pluralité de fois jusqu'à la dernière fois et que l'unité de détection d'état détecte que l'unité d'imagerie et l'unité d'éclairage ne se trouvent pas dans le liquide, le contrôleur (72) de mode d'observation est configuré pour arrêter l'opération d'imagerie suivante dans le mode d'observation à la lumière spéciale pour amener l'opération d'imagerie à s'exécuter dans le mode d'observation à la lumière ordinaire à la place du mode d'observation à la lumière spéciale.

**4.** Système (2) d'endoscope de type capsule selon la revendication 1, dans lequel
la source de lumière ordinaire (10) est une source de lumière blanche, et
la source de lumière spéciale (11) est une source de lumière à bande étroite qui émet une lumière spectrale d'une composante de couleur spécifique.

**5.** Système (2) d'endoscope de type capsule selon la revendication 4, dans lequel
la source de lumière à bande étroite est configurée pour émettre la lumière spéciale ayant le bleu et le vert comme composante de couleur spécifique.

**6.** Système (2) d'endoscope de type capsule selon la revendication 1, comprenant en outre :

une unité de transmission (55) configurée pour transmettre les images capturées par l'unité d'imagerie (52).

FIG.1

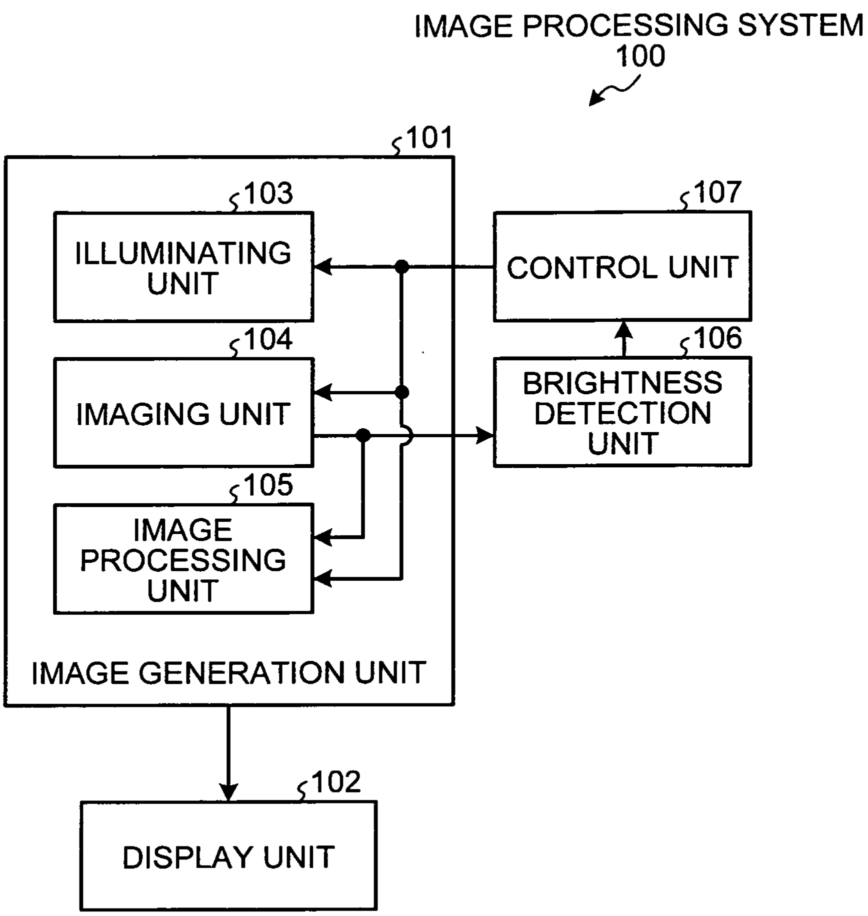
IMAGE PROCESSING SYSTEM
100
101
103
ILLUMINATING UNIT
107
CONTROL UNIT
104
106
IMAGING UNIT
BRIGHTNESS DETECTION UNIT
105
IMAGE PROCESSING UNIT
IMAGE GENERATION UNIT
102
DISPLAY UNIT

FIG.2

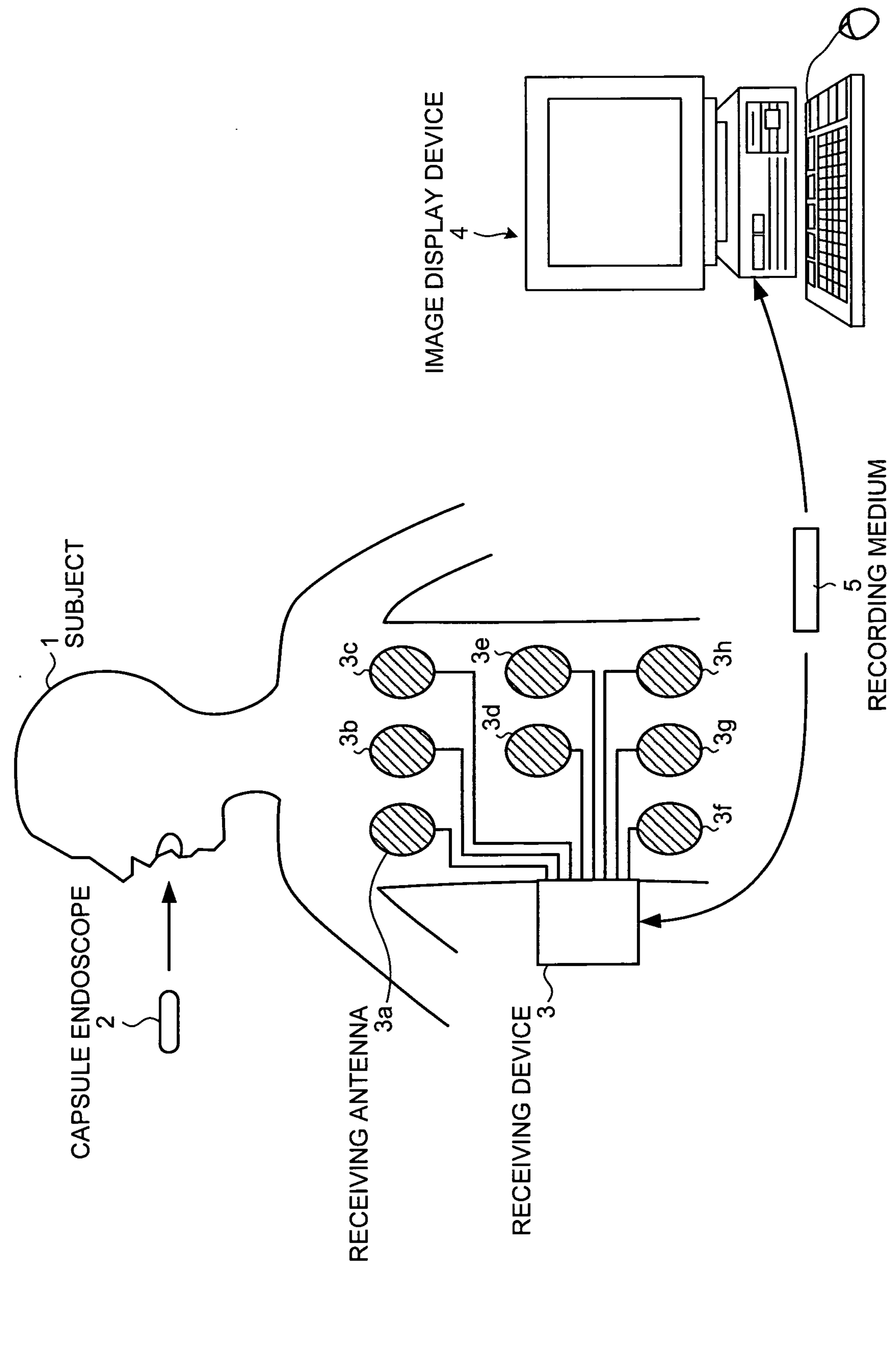
1
SUBJECT
CAPSULE ENDOSCOPE
2
RECEIVING ANTENNA
3a
3b
3c
3d
3e
3f
3g
3h
RECEIVING DEVICE
3
5
RECORDING MEDIUM
IMAGE DISPLAY DEVICE
4

FIG.3

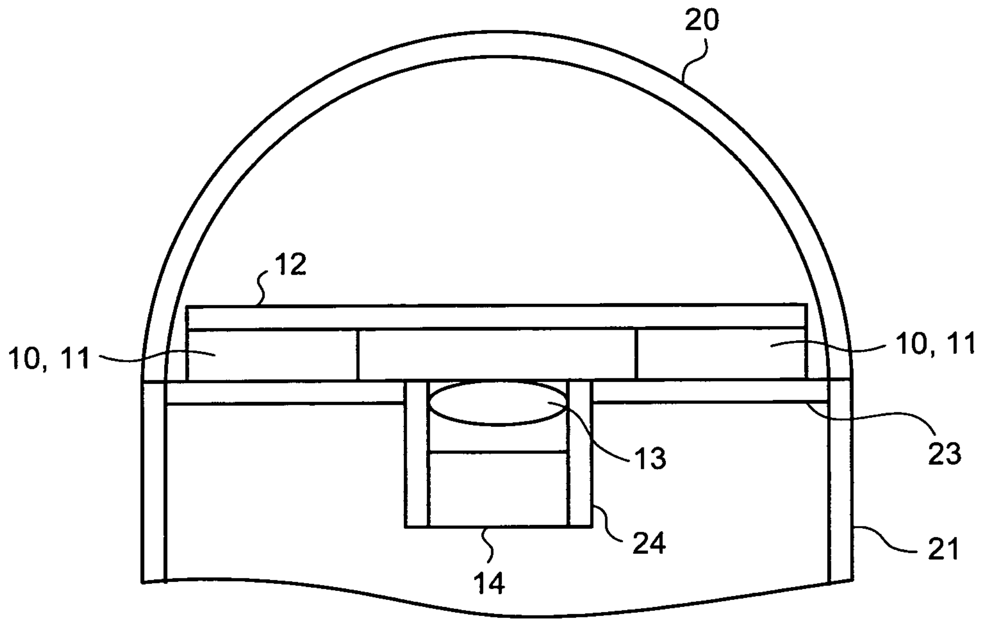


FIG.4

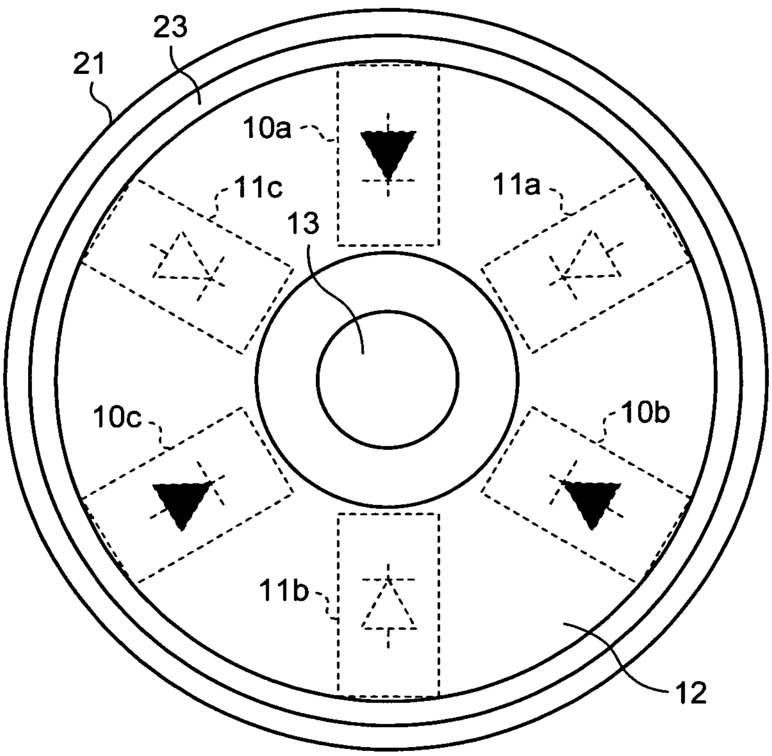

FIG.5

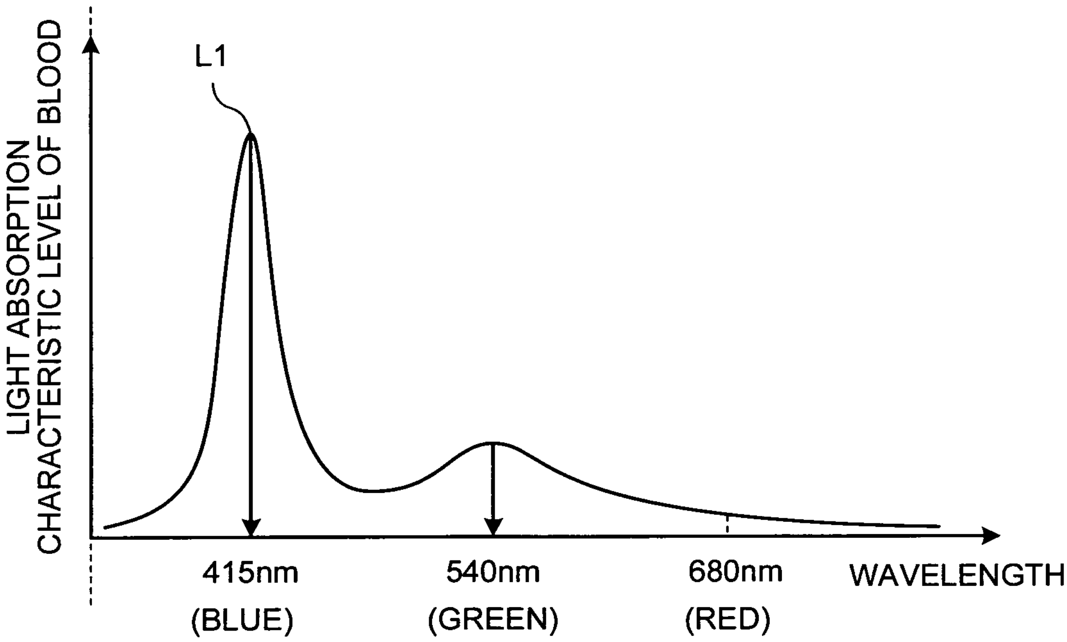
LIGHT ABSORPTION CHARACTERISTIC LEVEL OF BLOOD
L1
415nm
(BLUE)
540nm
(GREEN)
680nm
(RED)
WAVELENGTH

FIG.6

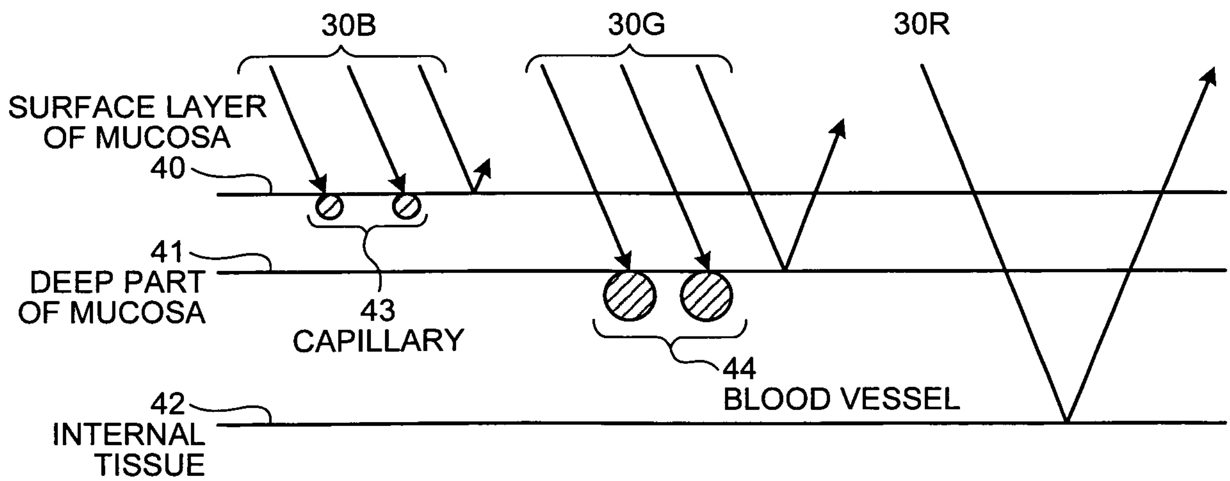
30B
30G
30R
SURFACE LAYER OF MUCOSA
40
41
DEEP PART OF MUCOSA
43
CAPILLARY
44
BLOOD VESSEL
42
INTERNAL TISSUE

FIG.7

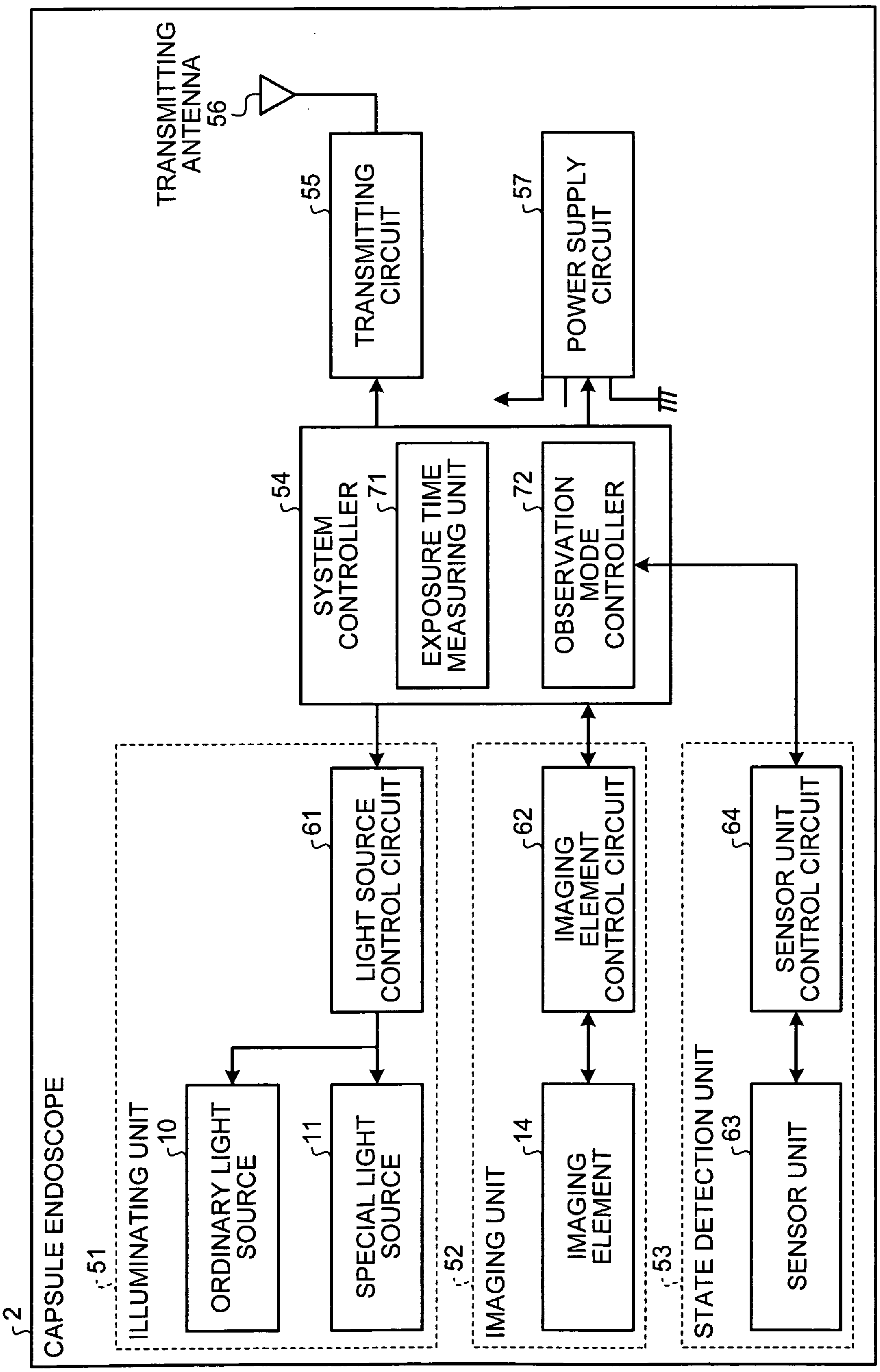
TRANSMITTING ANTENNA
56
55
TRANSMITTING CIRCUIT
57
POWER SUPPLY CIRCUIT
54
SYSTEM CONTROLLER
71
EXPOSURE TIME MEASURING UNIT
72
OBSERVATION MODE CONTROLLER
2
CAPSULE ENDOSCOPE
51
ILLUMINATING UNIT
10
ORDINARY LIGHT SOURCE
11
SPECIAL LIGHT SOURCE
61
LIGHT SOURCE CONTROL CIRCUIT
52
IMAGING UNIT
14
IMAGING ELEMENT
62
IMAGING ELEMENT CONTROL CIRCUIT
53
STATE DETECTION UNIT
63
SENSOR UNIT
64
SENSOR UNIT CONTROL CIRCUIT

FIG.8

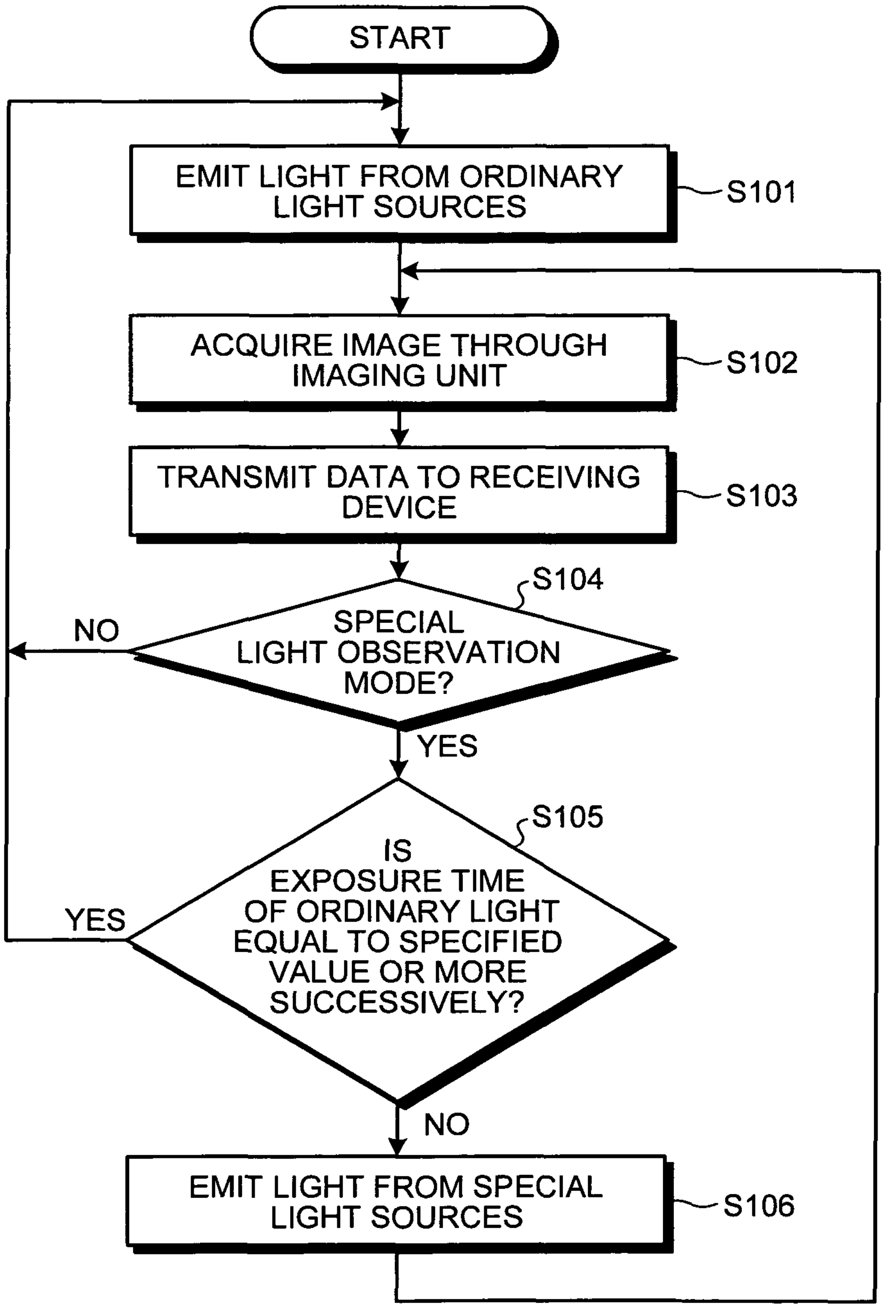
START
EMIT LIGHT FROM ORDINARY LIGHT SOURCES
S101
ACQUIRE IMAGE THROUGH IMAGING UNIT
S102
TRANSMIT DATA TO RECEIVING DEVICE
S103
S104
SPECIAL LIGHT OBSERVATION MODE?
NO
YES
S105
IS EXPOSURE TIME OF ORDINARY LIGHT EQUAL TO SPECIFIED VALUE OR MORE SUCCESSIVELY?
YES
NO
EMIT LIGHT FROM SPECIAL LIGHT SOURCES
S106

FIG.9

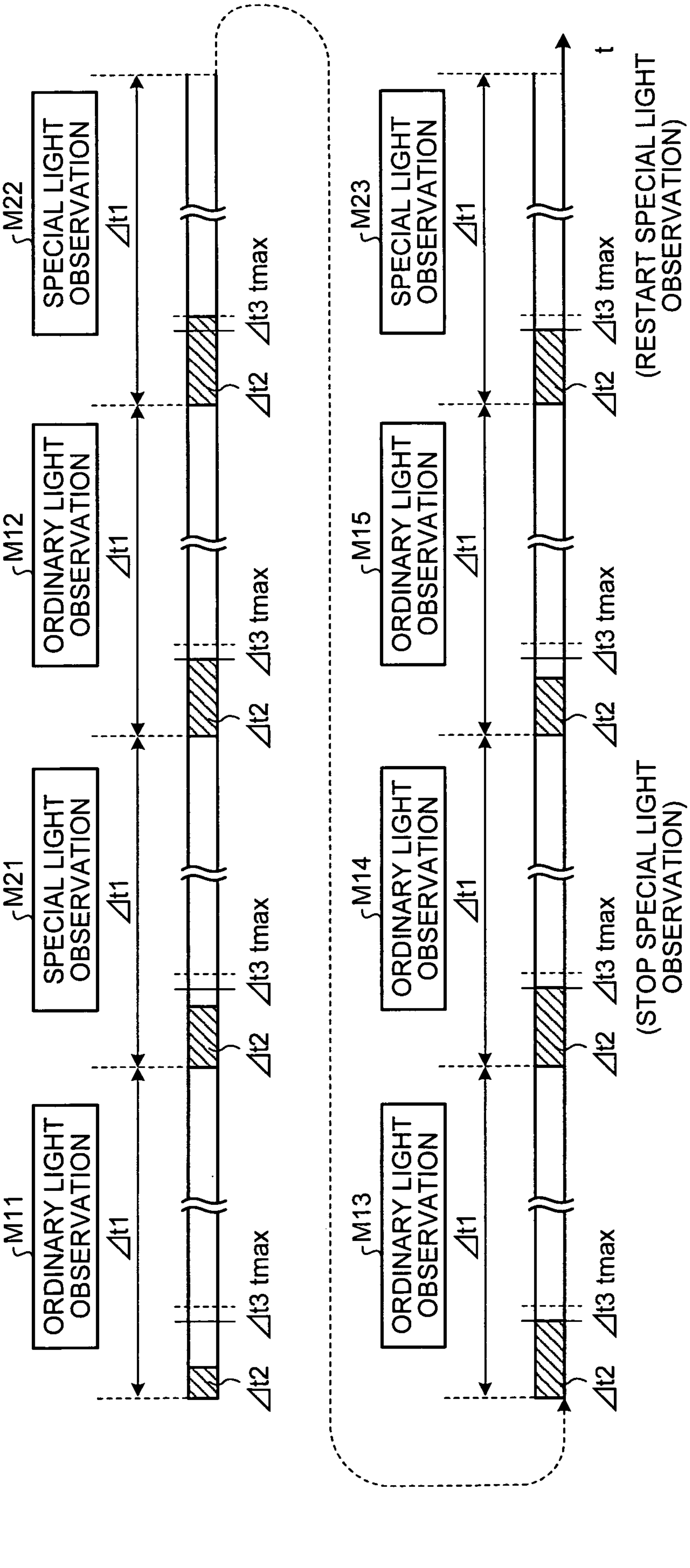
M11
ORDINARY LIGHT OBSERVATION
M21
SPECIAL LIGHT OBSERVATION
M12
ORDINARY LIGHT OBSERVATION
M22
SPECIAL LIGHT OBSERVATION
M13
ORDINARY LIGHT OBSERVATION
M14
ORDINARY LIGHT OBSERVATION
M15
ORDINARY LIGHT OBSERVATION
M23
SPECIAL LIGHT OBSERVATION
Δt1
Δt2
Δt3
tmax
t
(STOP SPECIAL LIGHT OBSERVATION)
(RESTART SPECIAL LIGHT OBSERVATION)

FIG.10

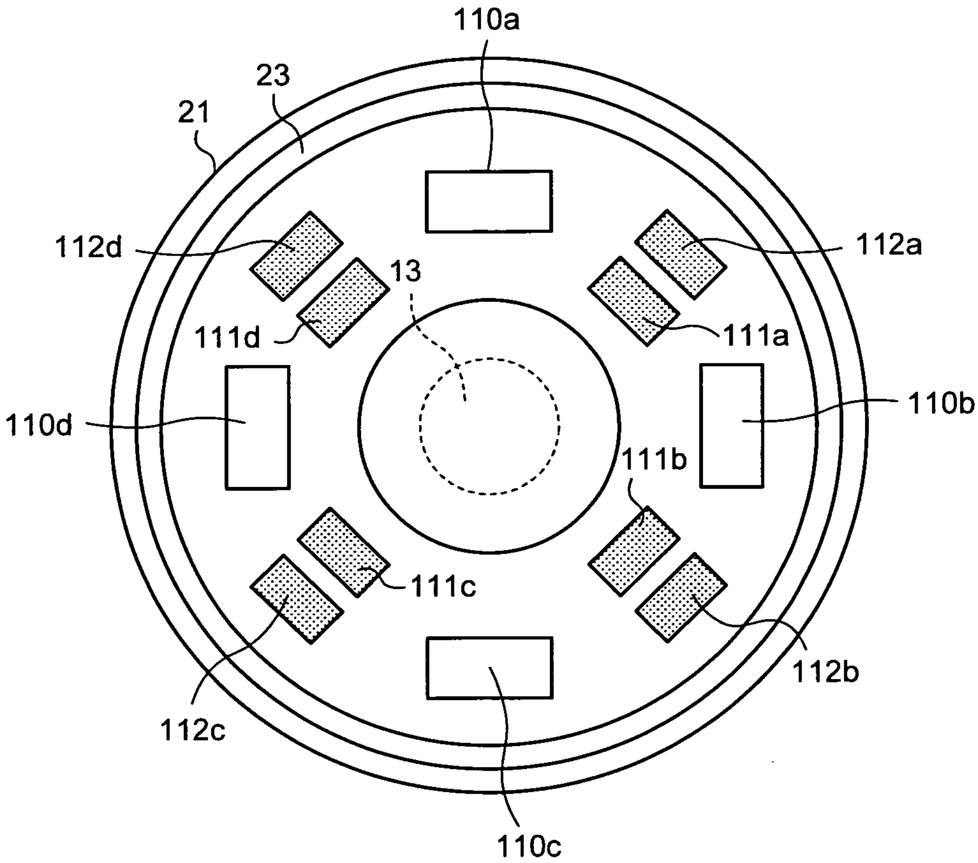
110a
23
21
112d
13
112a
111d
111a
110d
110b
111b
111c
112c
112b
110c

FIG.11

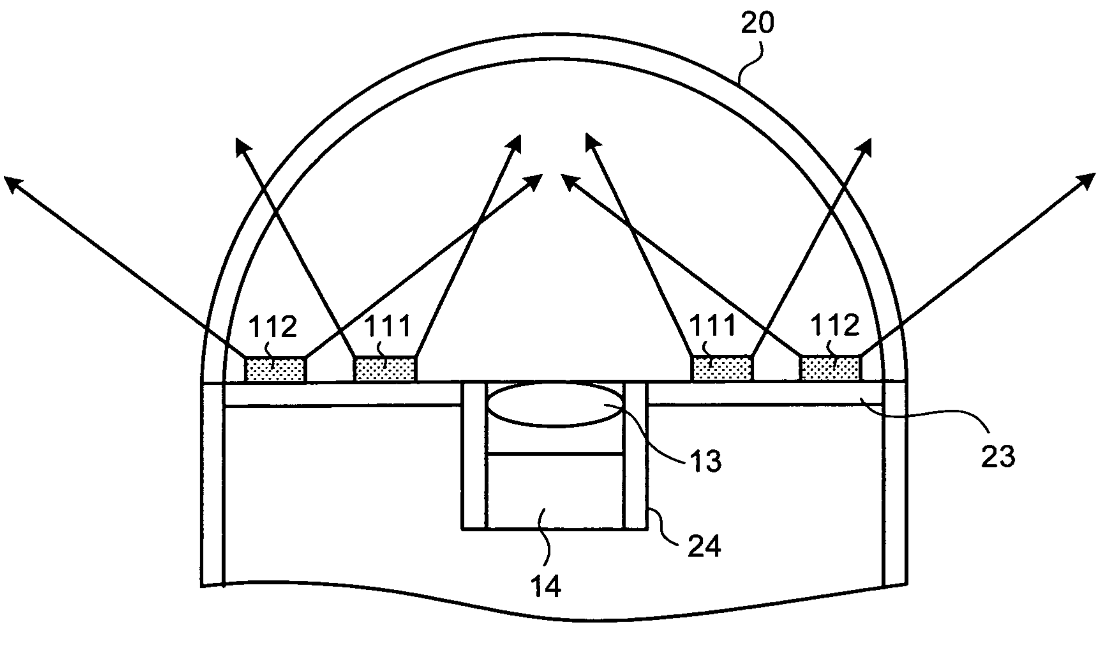
20
112
111
111
112
13
23
24
14

FIG.12

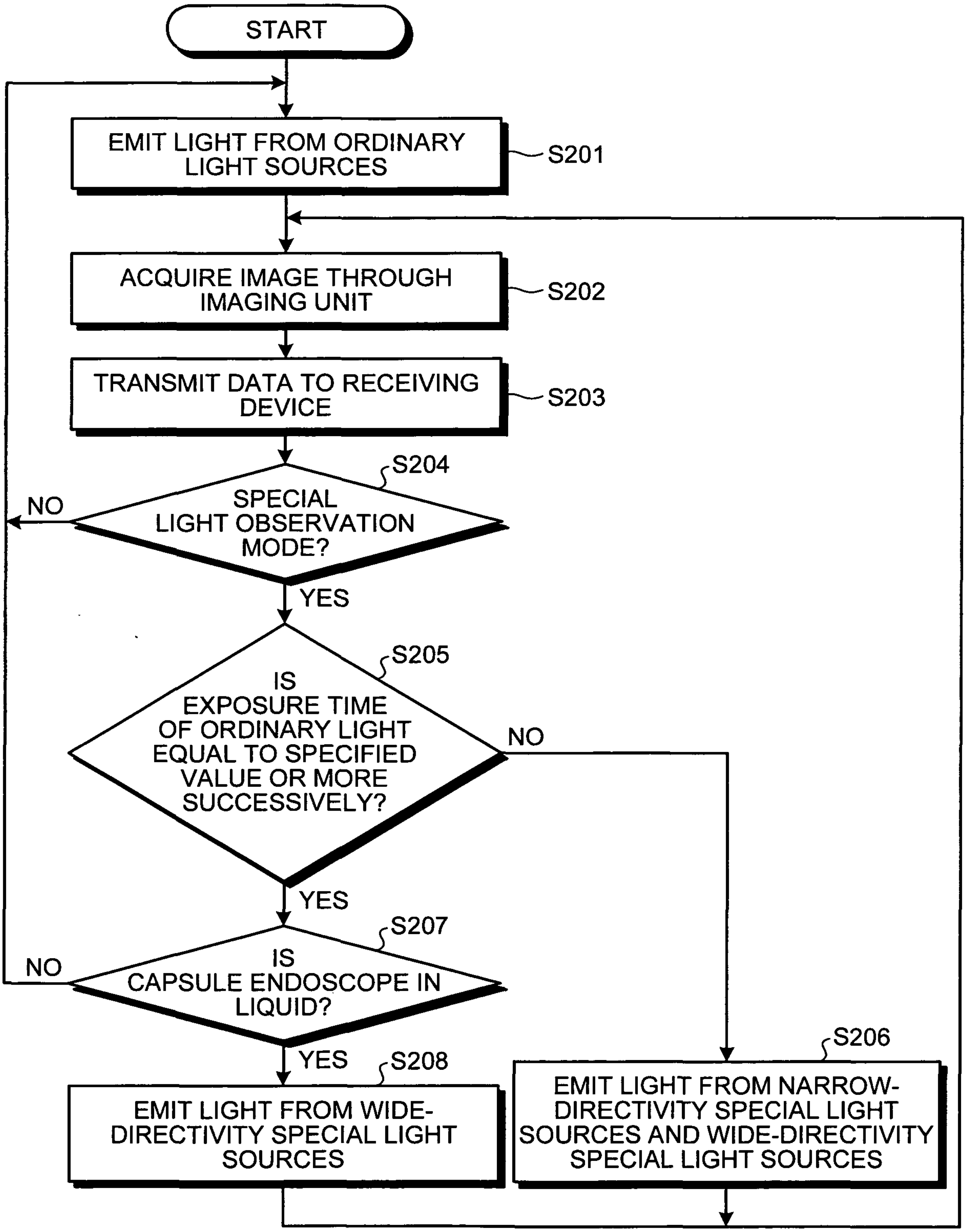
START
EMIT LIGHT FROM ORDINARY LIGHT SOURCES
S201
ACQUIRE IMAGE THROUGH IMAGING UNIT
S202
TRANSMIT DATA TO RECEIVING DEVICE
S203
S204
SPECIAL LIGHT OBSERVATION MODE?
NO
YES
S205
IS EXPOSURE TIME OF ORDINARY LIGHT EQUAL TO SPECIFIED VALUE OR MORE SUCCESSIVELY?
NO
YES
S207
IS CAPSULE ENDOSCOPE IN LIQUID?
NO
YES
S208
EMIT LIGHT FROM WIDE-DIRECTIVITY SPECIAL LIGHT SOURCES
S206
EMIT LIGHT FROM NARROW-DIRECTIVITY SPECIAL LIGHT SOURCES AND WIDE-DIRECTIVITY SPECIAL LIGHT SOURCES

FIG.13

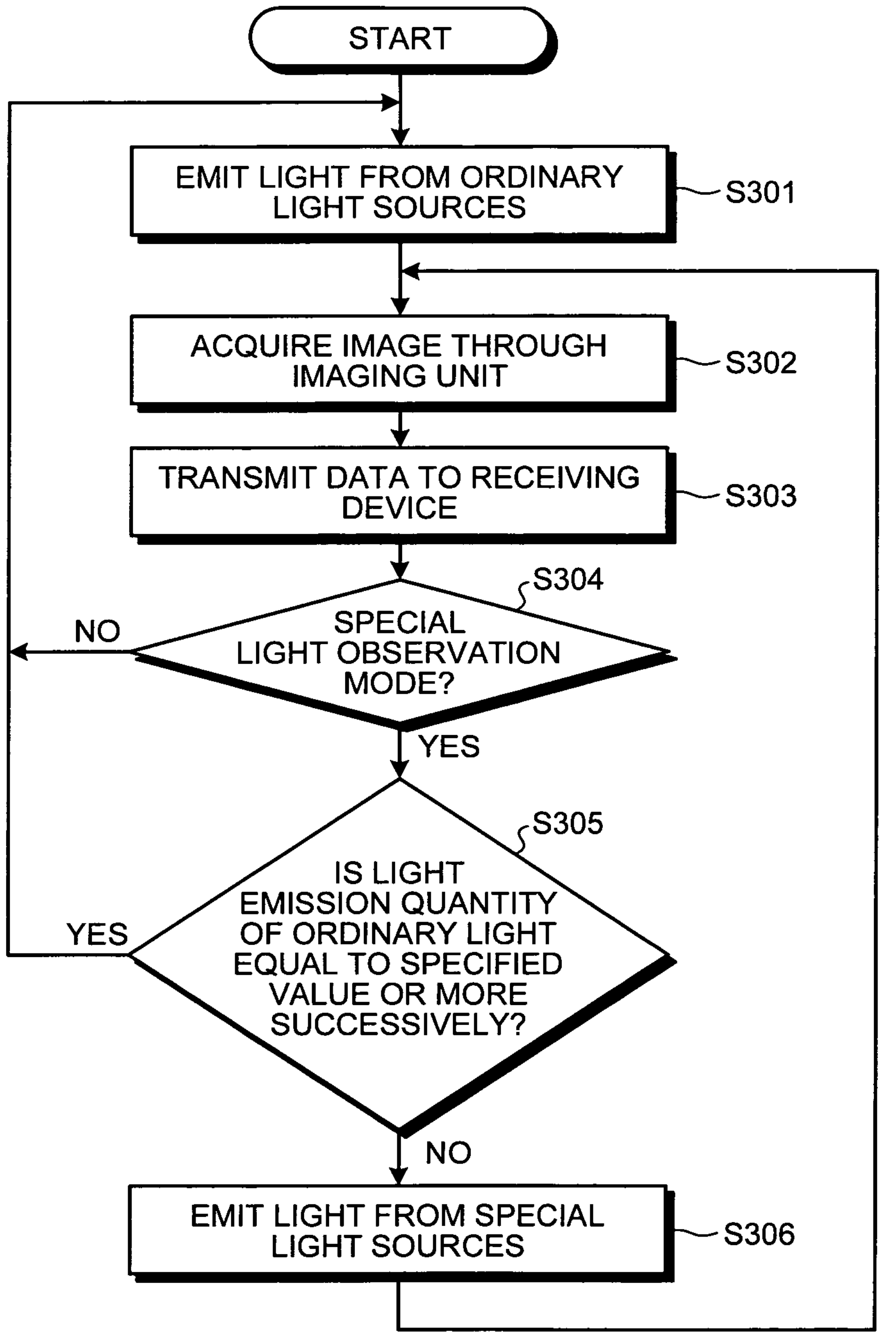
START
EMIT LIGHT FROM ORDINARY LIGHT SOURCES
S301
ACQUIRE IMAGE THROUGH IMAGING UNIT
S302
TRANSMIT DATA TO RECEIVING DEVICE
S303
S304
SPECIAL LIGHT OBSERVATION MODE?
NO
YES
S305
IS LIGHT EMISSION QUANTITY OF ORDINARY LIGHT EQUAL TO SPECIFIED VALUE OR MORE SUCCESSIVELY?
YES
NO
EMIT LIGHT FROM SPECIAL LIGHT SOURCES
S306

FIG.14

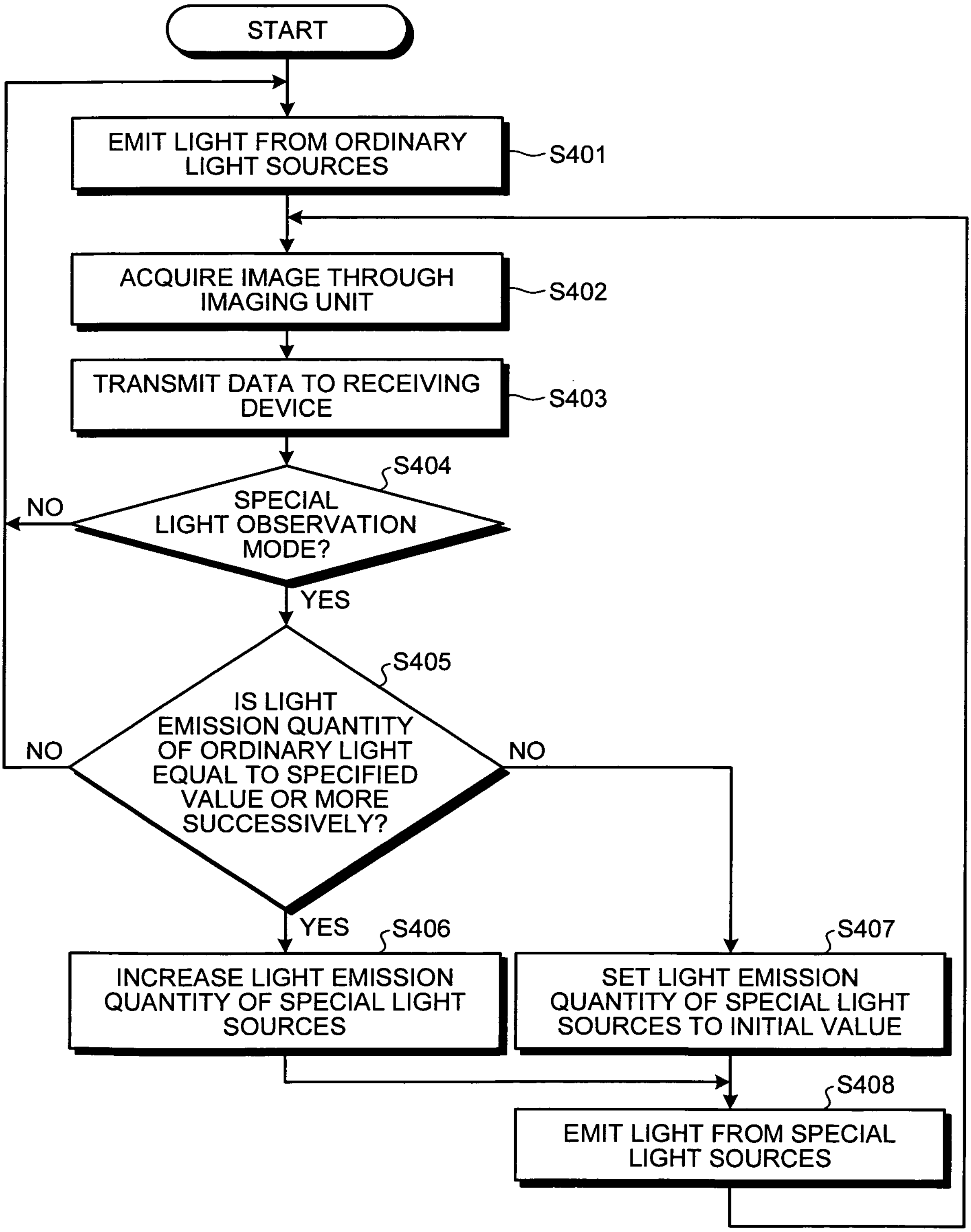
START
EMIT LIGHT FROM ORDINARY LIGHT SOURCES
S401
ACQUIRE IMAGE THROUGH IMAGING UNIT
S402
TRANSMIT DATA TO RECEIVING DEVICE
S403
SPECIAL LIGHT OBSERVATION MODE?
S404
NO
YES
IS LIGHT EMISSION QUANTITY OF ORDINARY LIGHT EQUAL TO SPECIFIED VALUE OR MORE SUCCESSIVELY?
S405
NO
NO
YES
S406
INCREASE LIGHT EMISSION QUANTITY OF SPECIAL LIGHT SOURCES
S407
SET LIGHT EMISSION QUANTITY OF SPECIAL LIGHT SOURCES TO INITIAL VALUE
S408
EMIT LIGHT FROM SPECIAL LIGHT SOURCES

FIG.15

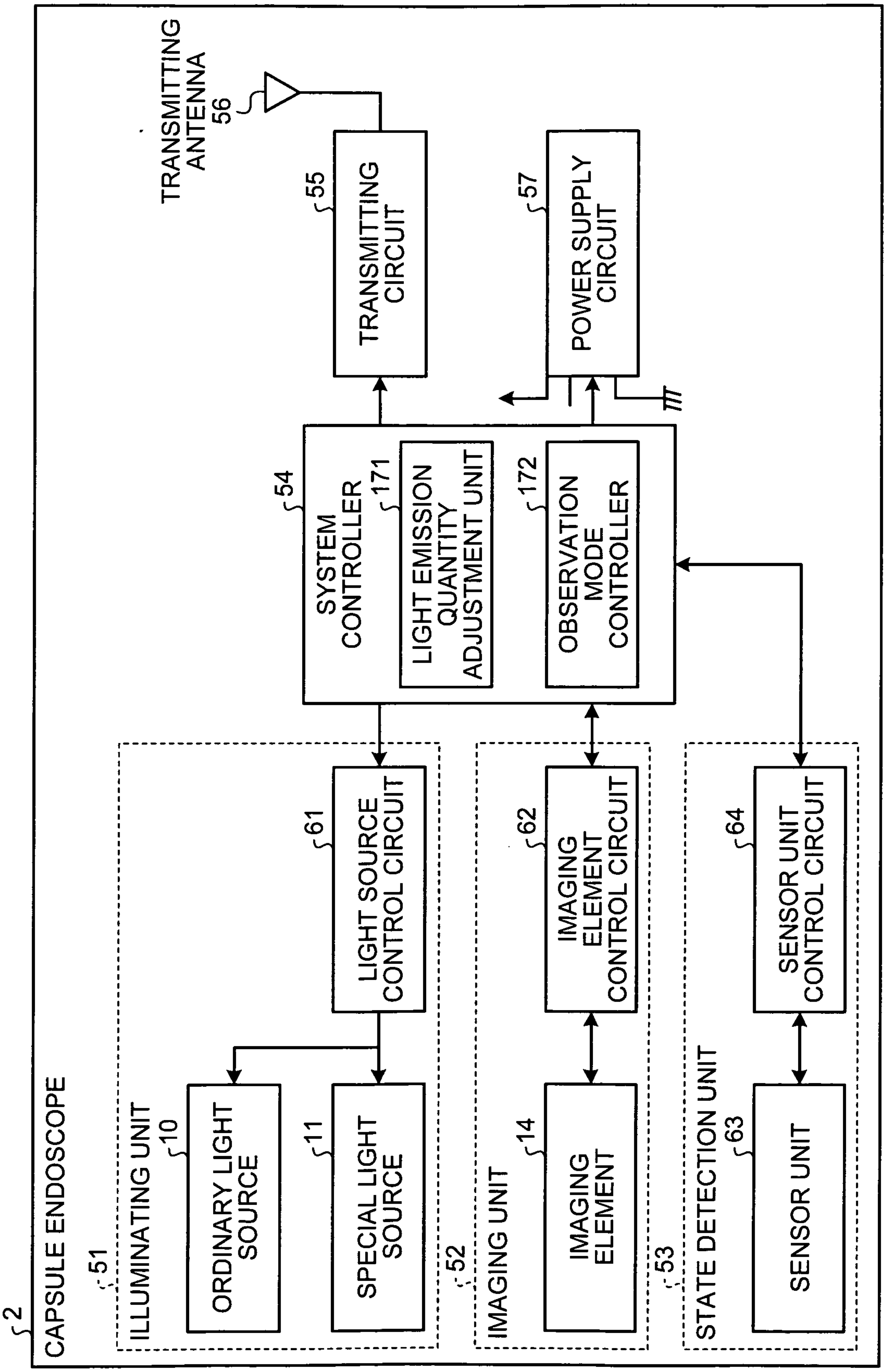
2
CAPSULE ENDOSCOPE
51
ILLUMINATING UNIT
10
ORDINARY LIGHT SOURCE
11
SPECIAL LIGHT SOURCE
61
LIGHT SOURCE CONTROL CIRCUIT
52
IMAGING UNIT
14
IMAGING ELEMENT
62
IMAGING ELEMENT CONTROL CIRCUIT
53
STATE DETECTION UNIT
63
SENSOR UNIT
64
SENSOR UNIT CONTROL CIRCUIT
54
SYSTEM CONTROLLER
171
LIGHT EMISSION QUANTITY ADJUSTMENT UNIT
172
OBSERVATION MODE CONTROLLER
55
TRANSMITTING CIRCUIT
TRANSMITTING ANTENNA
56
57
POWER SUPPLY CIRCUIT

FIG.16

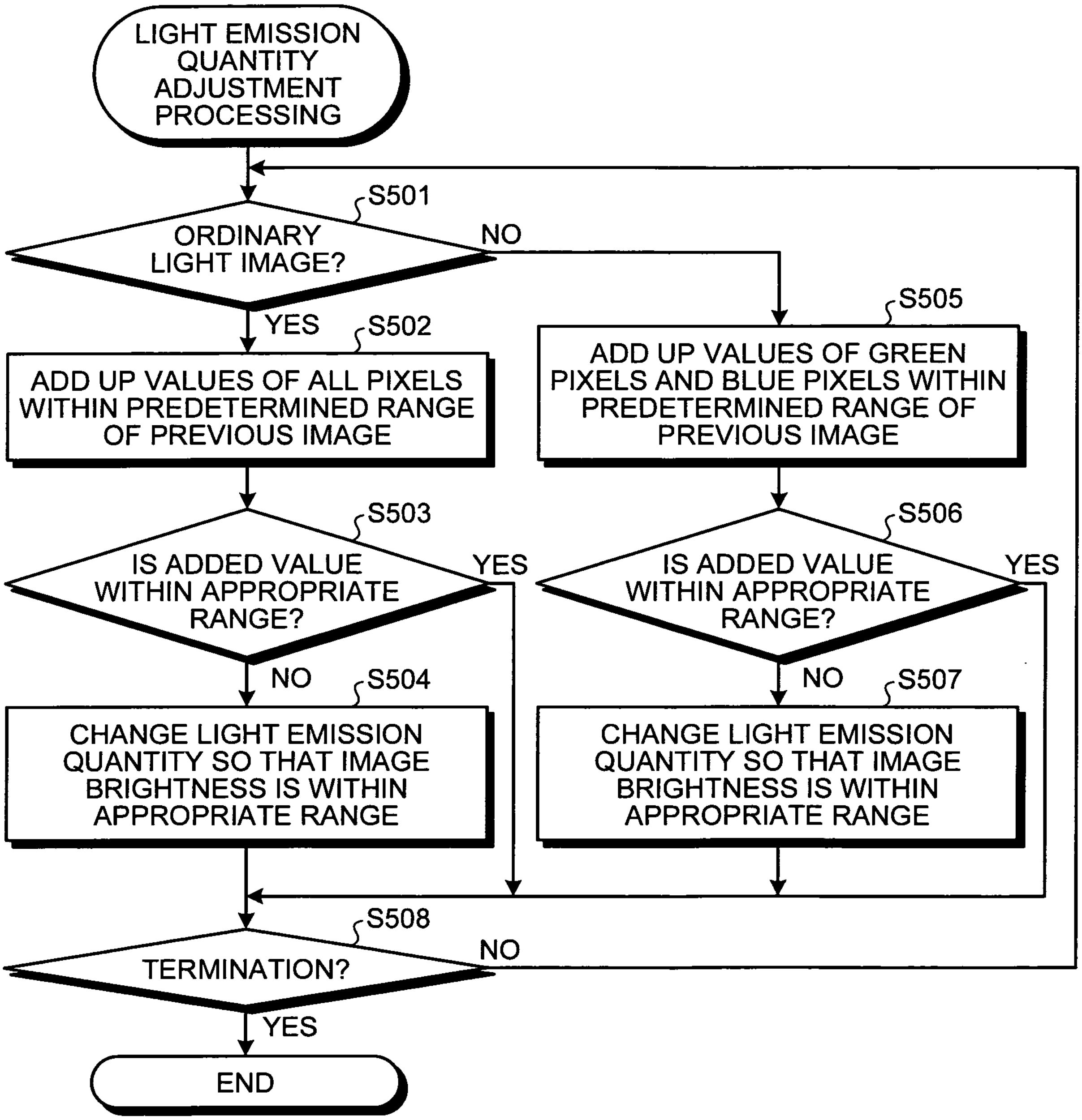
LIGHT EMISSION QUANTITY ADJUSTMENT PROCESSING
S501
ORDINARY LIGHT IMAGE?
NO
YES
S502
ADD UP VALUES OF ALL PIXELS WITHIN PREDETERMINED RANGE OF PREVIOUS IMAGE
S503
IS ADDED VALUE WITHIN APPROPRIATE RANGE?
YES
NO
S504
CHANGE LIGHT EMISSION QUANTITY SO THAT IMAGE BRIGHTNESS IS WITHIN APPROPRIATE RANGE
S505
ADD UP VALUES OF GREEN PIXELS AND BLUE PIXELS WITHIN PREDETERMINED RANGE OF PREVIOUS IMAGE
S506
IS ADDED VALUE WITHIN APPROPRIATE RANGE?
YES
NO
S507
CHANGE LIGHT EMISSION QUANTITY SO THAT IMAGE BRIGHTNESS IS WITHIN APPROPRIATE RANGE
S508
TERMINATION?
NO
YES
END

FIG.17

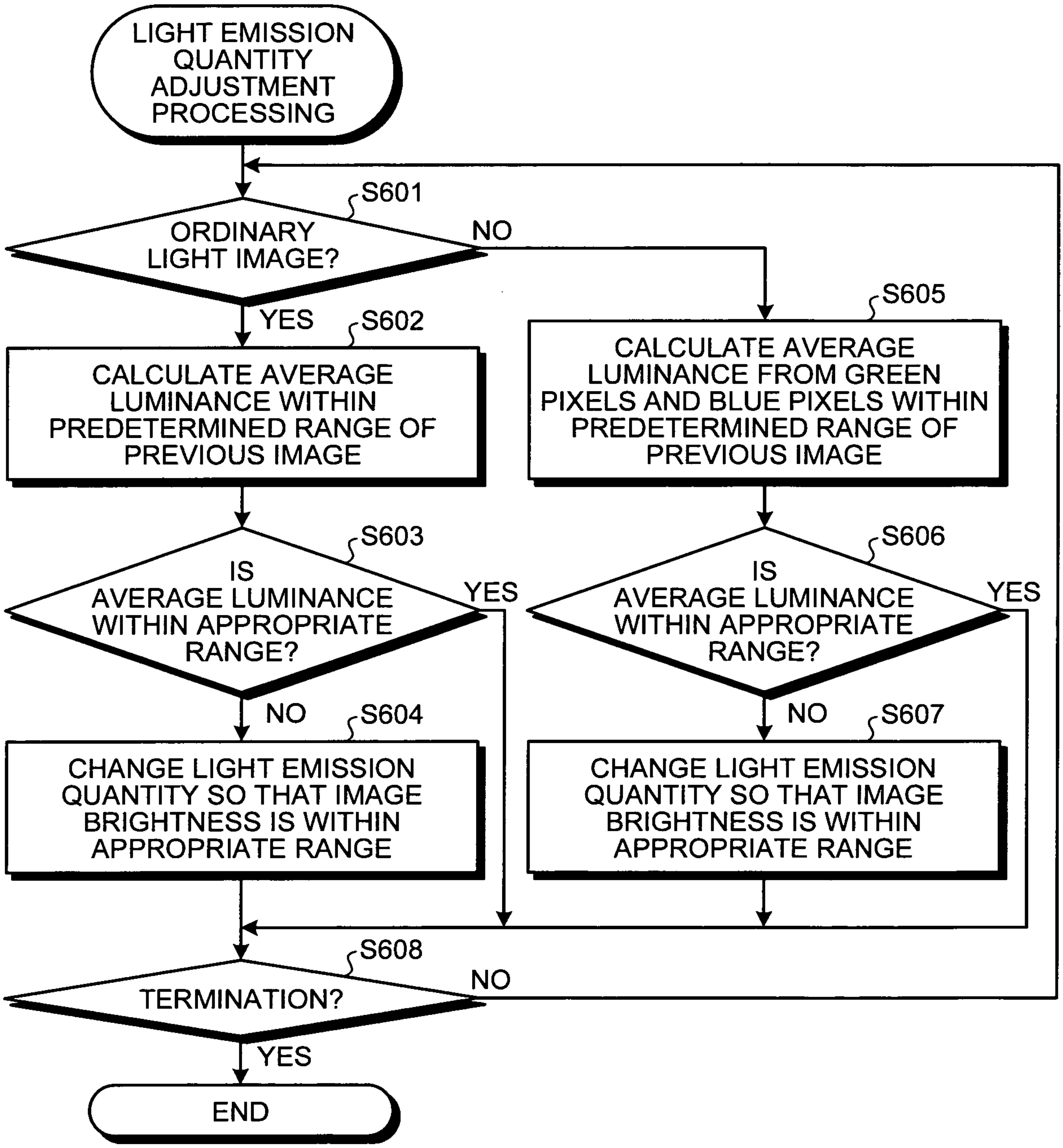
LIGHT EMISSION QUANTITY ADJUSTMENT PROCESSING
S601
ORDINARY LIGHT IMAGE?
NO
YES
S602
CALCULATE AVERAGE LUMINANCE WITHIN PREDETERMINED RANGE OF PREVIOUS IMAGE
S605
CALCULATE AVERAGE LUMINANCE FROM GREEN PIXELS AND BLUE PIXELS WITHIN PREDETERMINED RANGE OF PREVIOUS IMAGE
S603
IS AVERAGE LUMINANCE WITHIN APPROPRIATE RANGE?
YES
NO
S604
CHANGE LIGHT EMISSION QUANTITY SO THAT IMAGE BRIGHTNESS IS WITHIN APPROPRIATE RANGE
S606
IS AVERAGE LUMINANCE WITHIN APPROPRIATE RANGE?
YES
NO
S607
CHANGE LIGHT EMISSION QUANTITY SO THAT IMAGE BRIGHTNESS IS WITHIN APPROPRIATE RANGE
S608
TERMINATION?
NO
YES
END

FIG.18

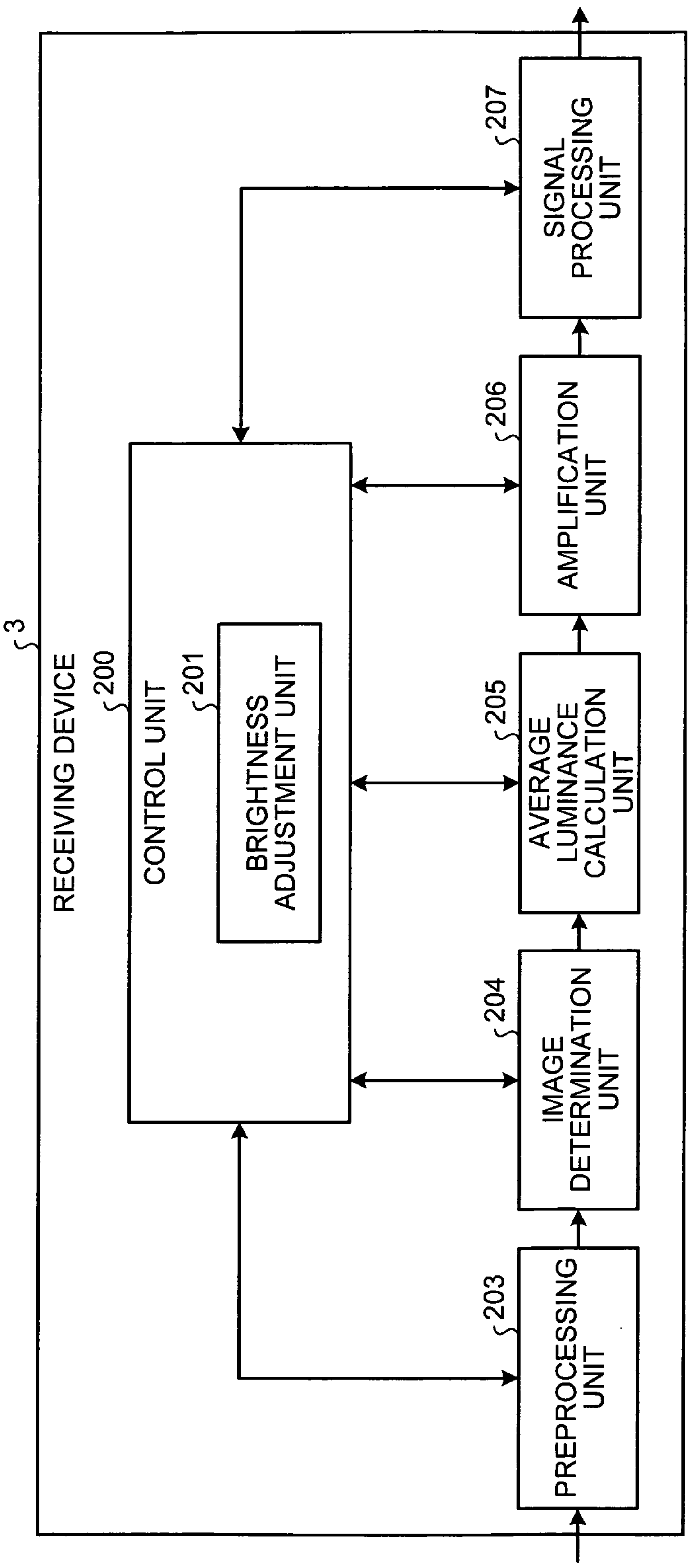
3
RECEIVING DEVICE
200
CONTROL UNIT
201
BRIGHTNESS ADJUSTMENT UNIT
203
PREPROCESSING UNIT
204
IMAGE DETERMINATION UNIT
205
AVERAGE LUMINANCE CALCULATION UNIT
206
AMPLIFICATION UNIT
207
SIGNAL PROCESSING UNIT

FIG.19

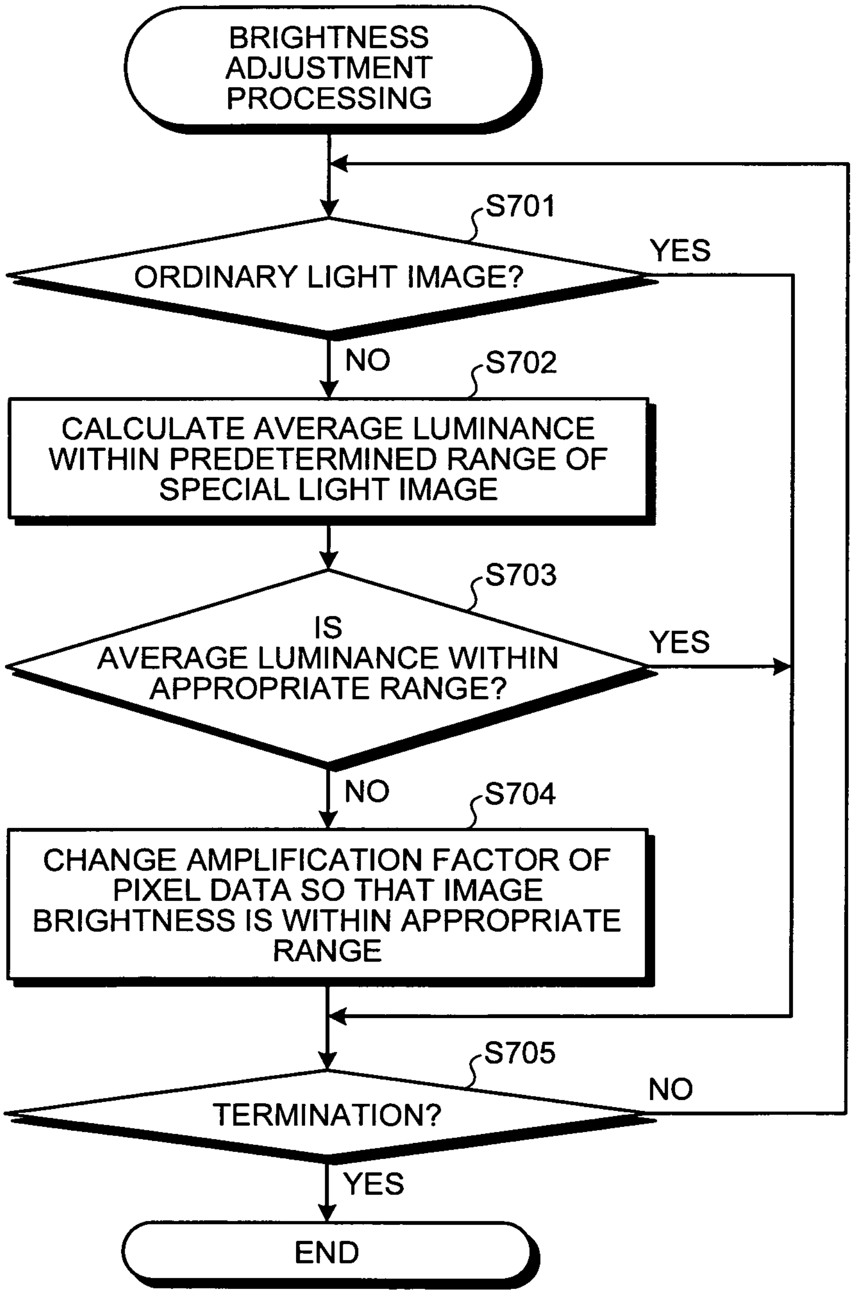
BRIGHTNESS ADJUSTMENT PROCESSING
S701
ORDINARY LIGHT IMAGE?
YES
NO
S702
CALCULATE AVERAGE LUMINANCE WITHIN PREDETERMINED RANGE OF SPECIAL LIGHT IMAGE
S703
IS AVERAGE LUMINANCE WITHIN APPROPRIATE RANGE?
YES
NO
S704
CHANGE AMPLIFICATION FACTOR OF PIXEL DATA SO THAT IMAGE BRIGHTNESS IS WITHIN APPROPRIATE RANGE
S705
TERMINATION?
NO
YES
END

FIG.20

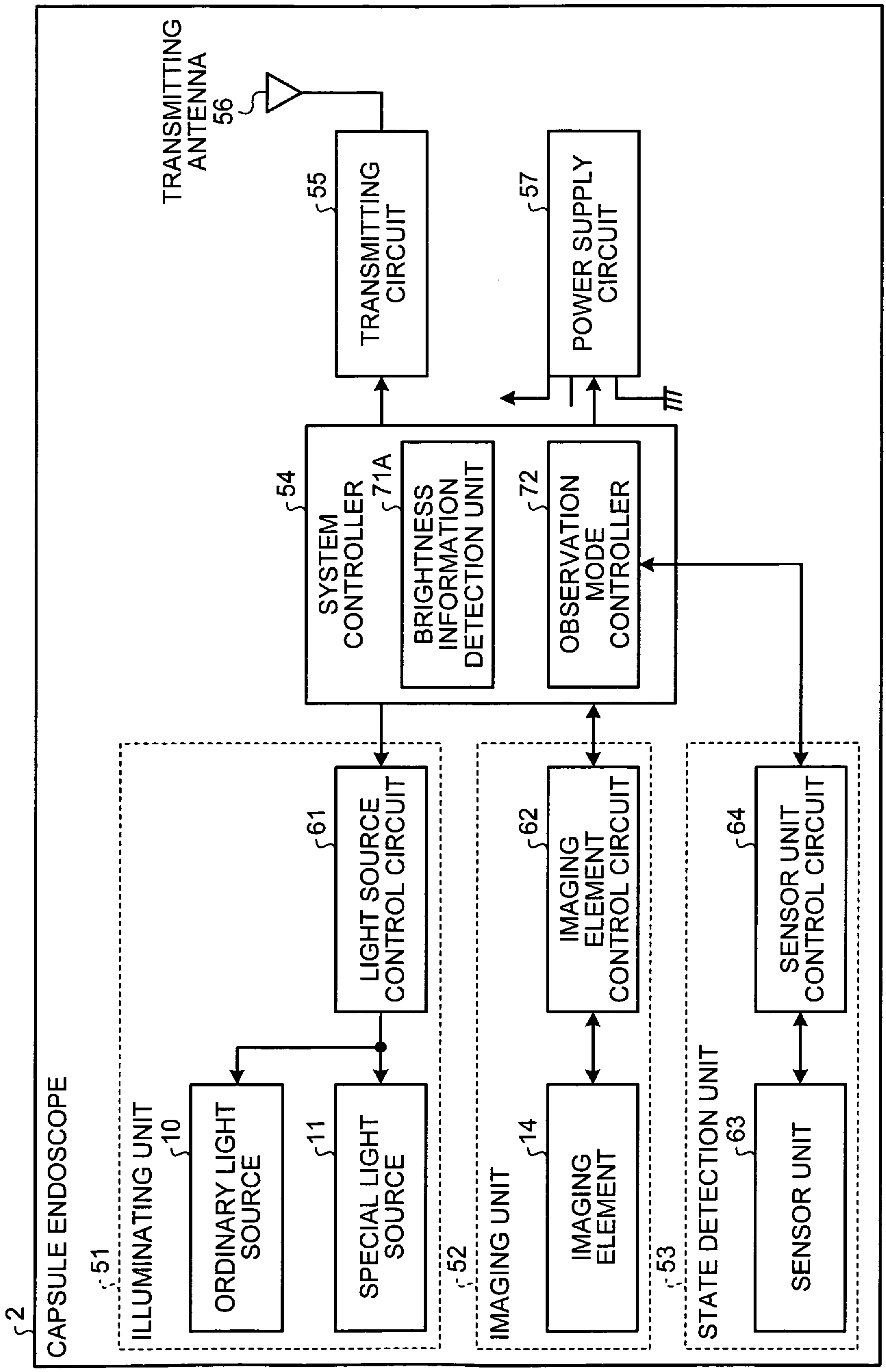
2
CAPSULE ENDOSCOPE
51
ILLUMINATING UNIT
10
ORDINARY LIGHT SOURCE
11
SPECIAL LIGHT SOURCE
61
LIGHT SOURCE CONTROL CIRCUIT
52
IMAGING UNIT
14
IMAGING ELEMENT
62
IMAGING ELEMENT CONTROL CIRCUIT
53
STATE DETECTION UNIT
63
SENSOR UNIT
64
SENSOR UNIT CONTROL CIRCUIT
54
SYSTEM CONTROLLER
71A
BRIGHTNESS INFORMATION DETECTION UNIT
72
OBSERVATION MODE CONTROLLER
55
TRANSMITTING CIRCUIT
TRANSMITTING ANTENNA
56
57
POWER SUPPLY CIRCUIT

FIG.21

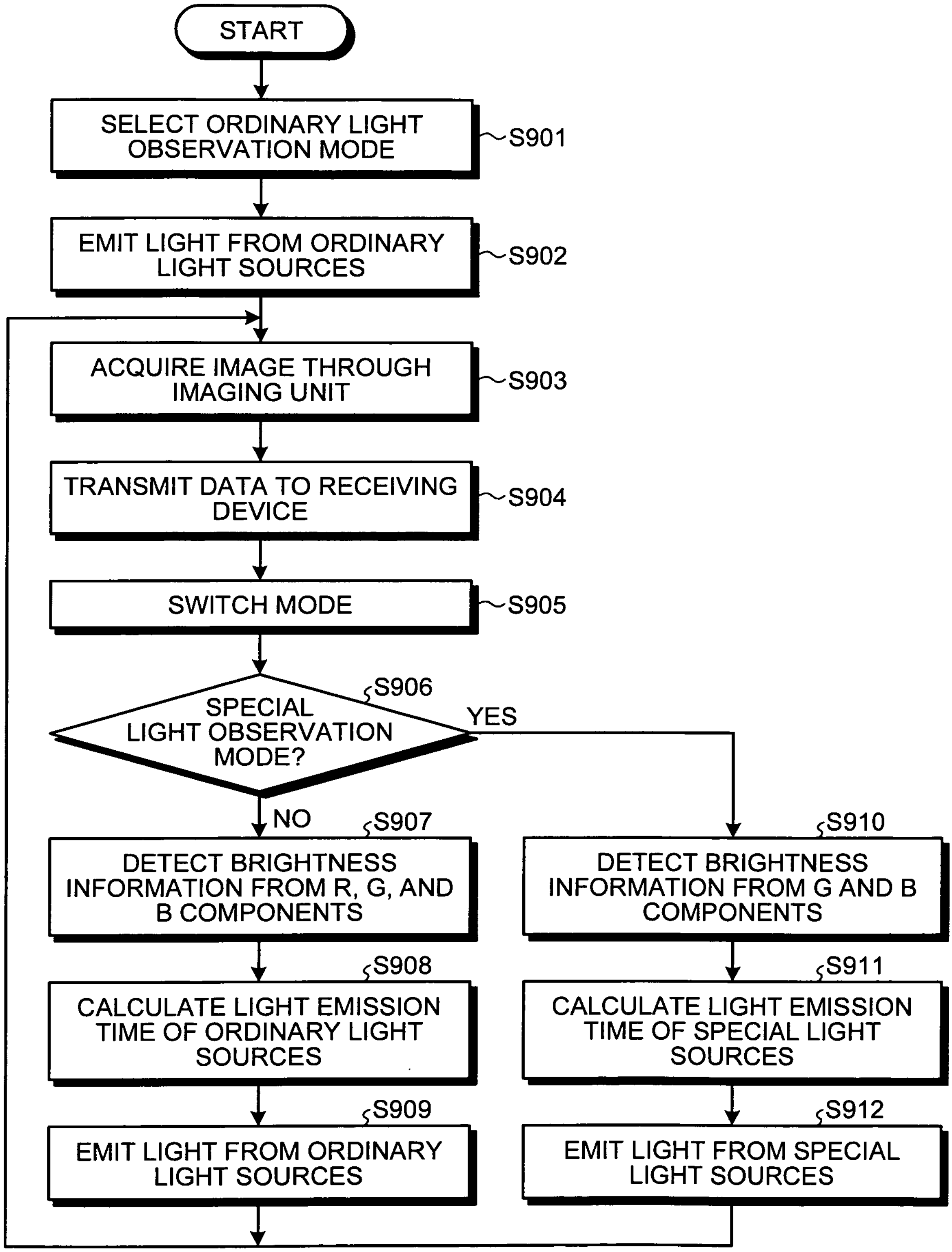
START
SELECT ORDINARY LIGHT OBSERVATION MODE
S901
EMIT LIGHT FROM ORDINARY LIGHT SOURCES
S902
ACQUIRE IMAGE THROUGH IMAGING UNIT
S903
TRANSMIT DATA TO RECEIVING DEVICE
S904
SWITCH MODE
S905
SPECIAL LIGHT OBSERVATION MODE?
S906
YES
NO
S907
DETECT BRIGHTNESS INFORMATION FROM R, G, AND B COMPONENTS
S908
CALCULATE LIGHT EMISSION TIME OF ORDINARY LIGHT SOURCES
S909
EMIT LIGHT FROM ORDINARY LIGHT SOURCES
S910
DETECT BRIGHTNESS INFORMATION FROM G AND B COMPONENTS
S911
CALCULATE LIGHT EMISSION TIME OF SPECIAL LIGHT SOURCES
S912
EMIT LIGHT FROM SPECIAL LIGHT SOURCES

FIG.22

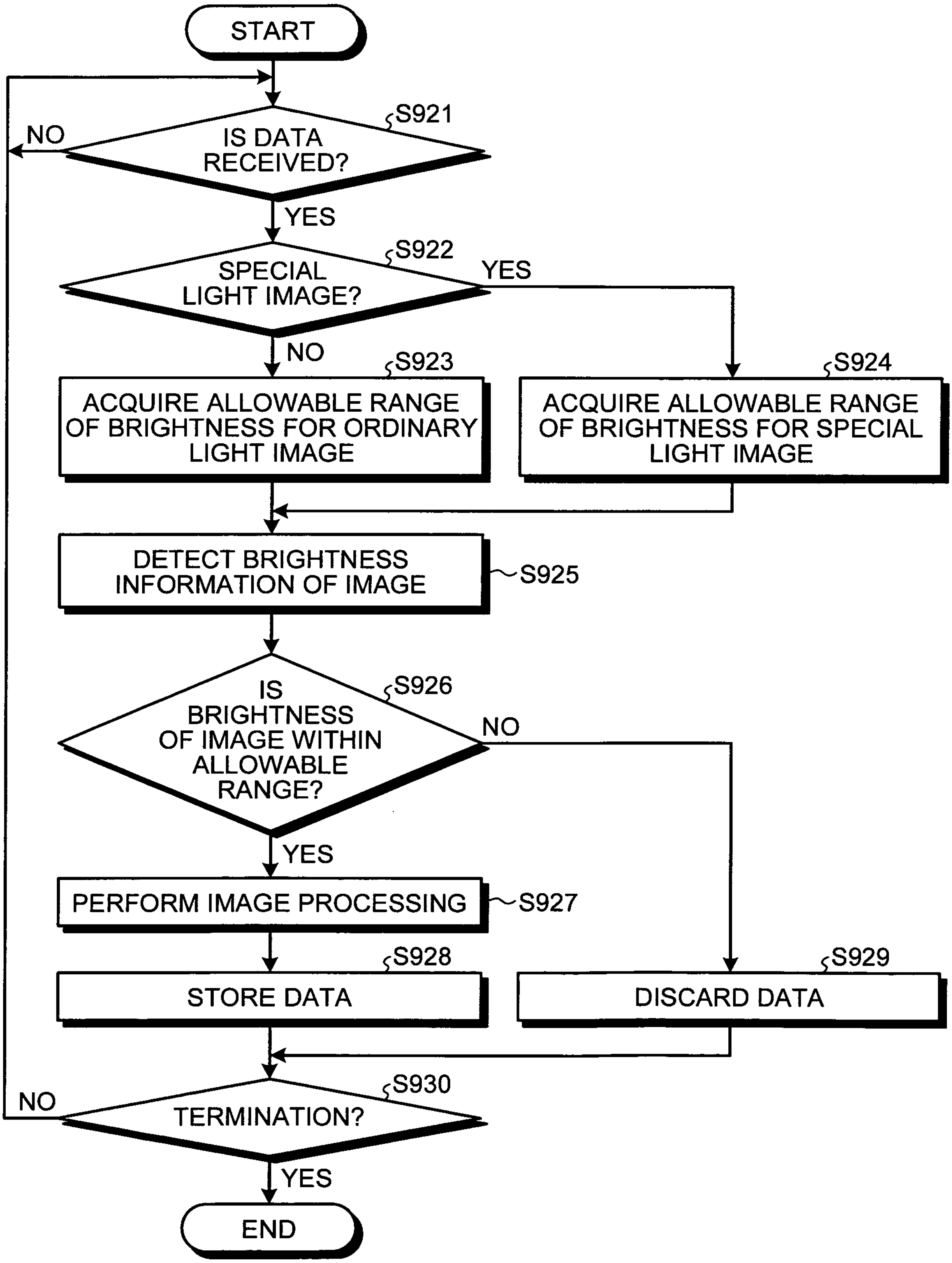
START
IS DATA RECEIVED?
S921
NO
YES
SPECIAL LIGHT IMAGE?
S922
YES
NO
ACQUIRE ALLOWABLE RANGE OF BRIGHTNESS FOR ORDINARY LIGHT IMAGE
S923
ACQUIRE ALLOWABLE RANGE OF BRIGHTNESS FOR SPECIAL LIGHT IMAGE
S924
DETECT BRIGHTNESS INFORMATION OF IMAGE
S925
IS BRIGHTNESS OF IMAGE WITHIN ALLOWABLE RANGE?
S926
NO
YES
PERFORM IMAGE PROCESSING
S927
STORE DATA
S928
DISCARD DATA
S929
TERMINATION?
S930
NO
YES
END

FIG.23

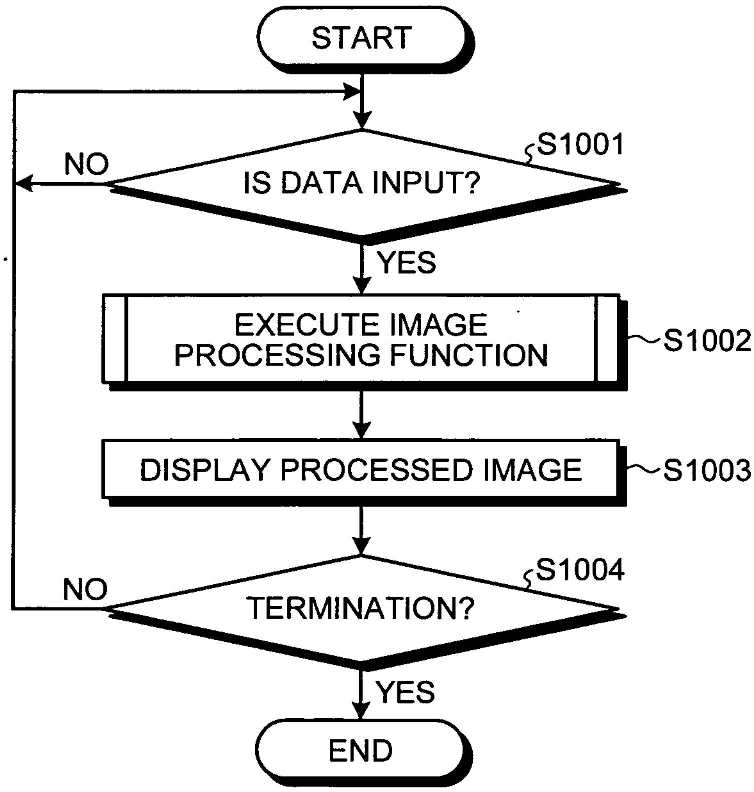
START
IS DATA INPUT?
S1001
NO
YES
EXECUTE IMAGE PROCESSING FUNCTION
S1002
DISPLAY PROCESSED IMAGE
S1003
TERMINATION?
S1004
NO
YES
END

FIG.24

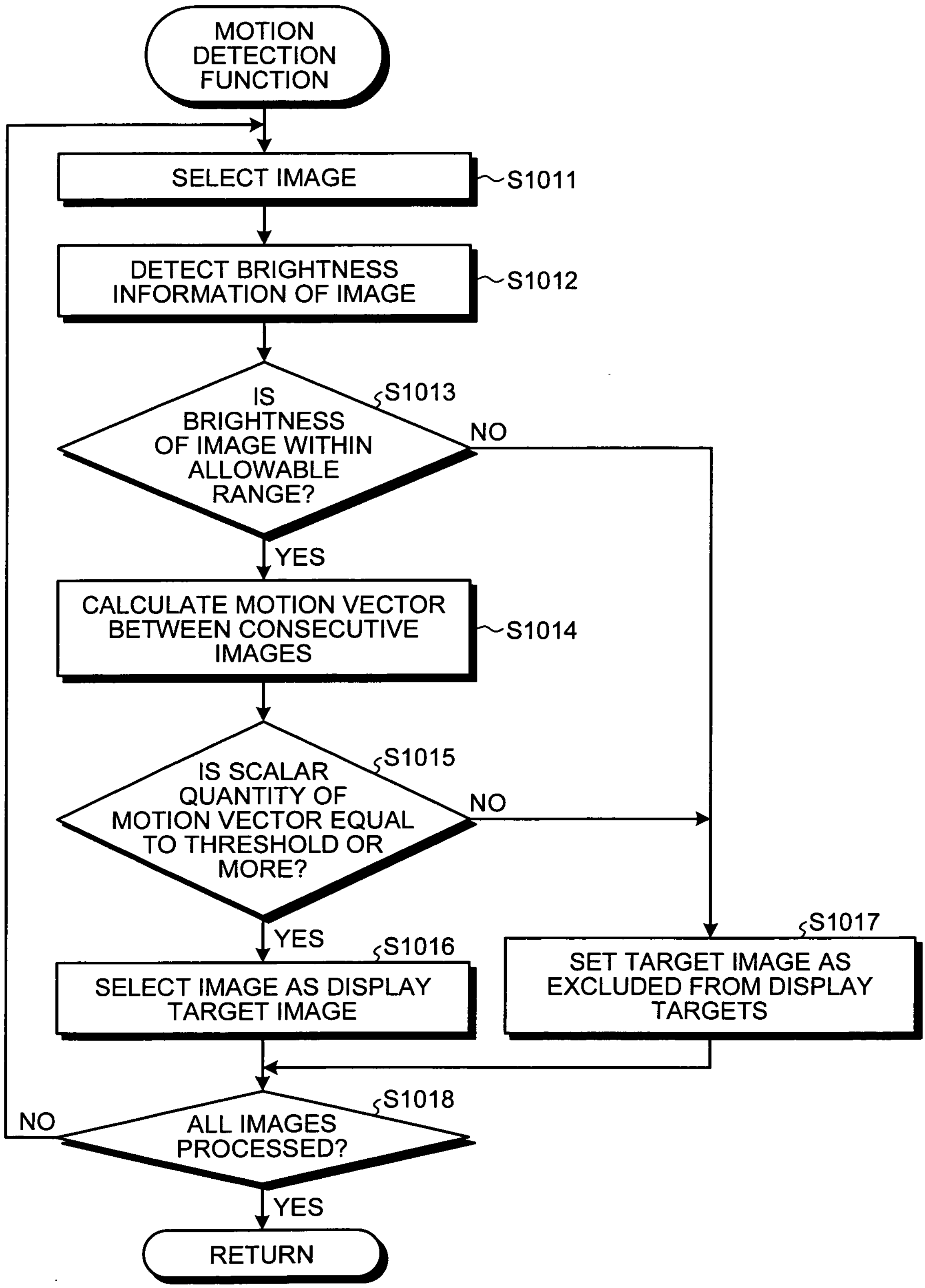
MOTION DETECTION FUNCTION
SELECT IMAGE
S1011
DETECT BRIGHTNESS INFORMATION OF IMAGE
S1012
IS BRIGHTNESS OF IMAGE WITHIN ALLOWABLE RANGE?
S1013
NO
YES
CALCULATE MOTION VECTOR BETWEEN CONSECUTIVE IMAGES
S1014
IS SCALAR QUANTITY OF MOTION VECTOR EQUAL TO THRESHOLD OR MORE?
S1015
NO
YES
SELECT IMAGE AS DISPLAY TARGET IMAGE
S1016
SET TARGET IMAGE AS EXCLUDED FROM DISPLAY TARGETS
S1017
ALL IMAGES PROCESSED?
S1018
NO
YES
RETURN

FIG.25

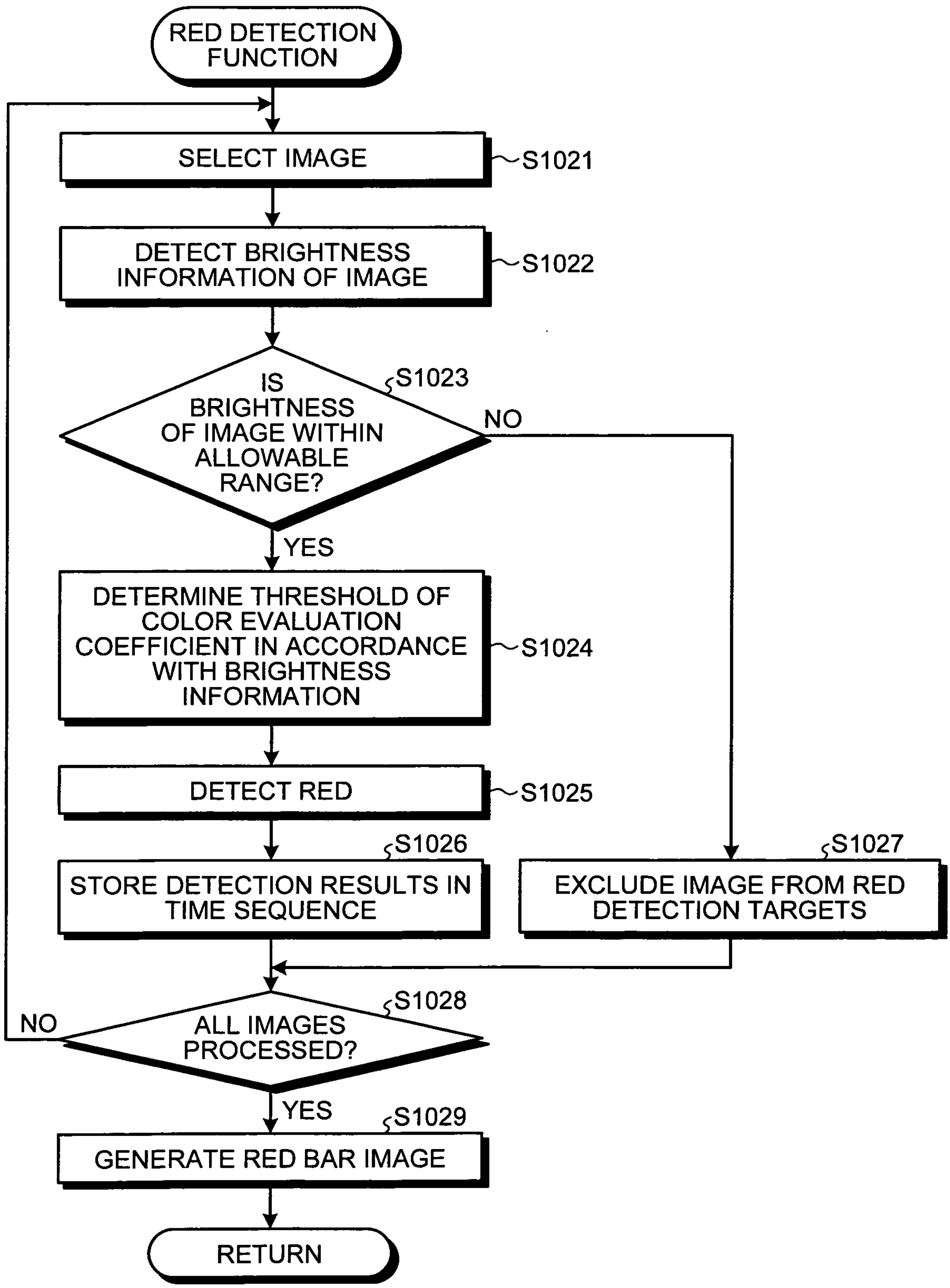
RED DETECTION FUNCTION
SELECT IMAGE
S1021
DETECT BRIGHTNESS INFORMATION OF IMAGE
S1022
IS BRIGHTNESS OF IMAGE WITHIN ALLOWABLE RANGE?
S1023
NO
YES
DETERMINE THRESHOLD OF COLOR EVALUATION COEFFICIENT IN ACCORDANCE WITH BRIGHTNESS INFORMATION
S1024
DETECT RED
S1025
S1026
STORE DETECTION RESULTS IN TIME SEQUENCE
S1027
EXCLUDE IMAGE FROM RED DETECTION TARGETS
S1028
NO
ALL IMAGES PROCESSED?
YES
S1029
GENERATE RED BAR IMAGE
RETURN

FIG.26

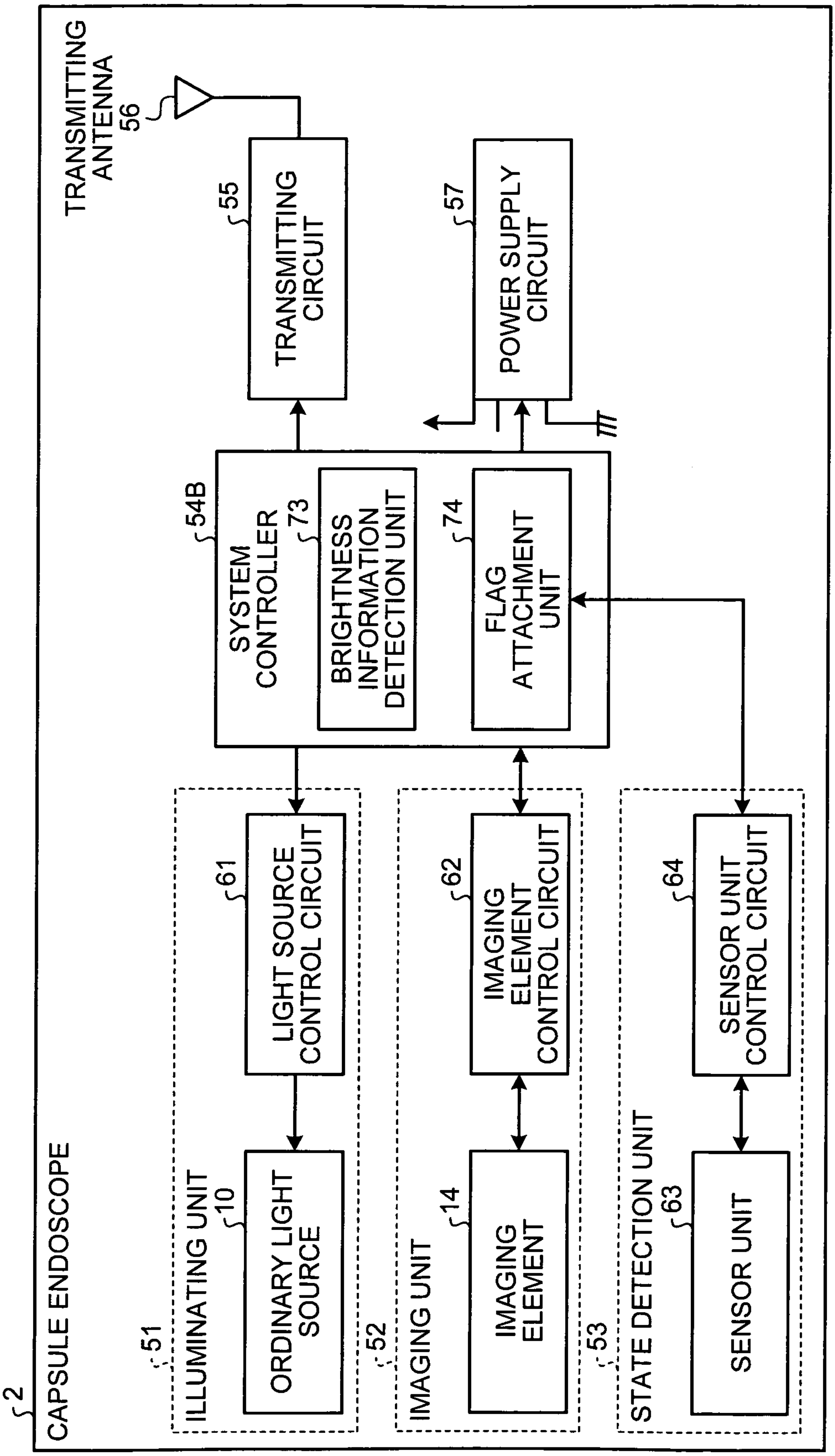
2
CAPSULE ENDOSCOPE
51
ILLUMINATING UNIT
10
ORDINARY LIGHT SOURCE
61
LIGHT SOURCE CONTROL CIRCUIT
52
IMAGING UNIT
14
IMAGING ELEMENT
62
IMAGING ELEMENT CONTROL CIRCUIT
53
STATE DETECTION UNIT
63
SENSOR UNIT
64
SENSOR UNIT CONTROL CIRCUIT
54B
SYSTEM CONTROLLER
73
BRIGHTNESS INFORMATION DETECTION UNIT
74
FLAG ATTACHMENT UNIT
55
TRANSMITTING CIRCUIT
56
TRANSMITTING ANTENNA
57
POWER SUPPLY CIRCUIT

FIG.27

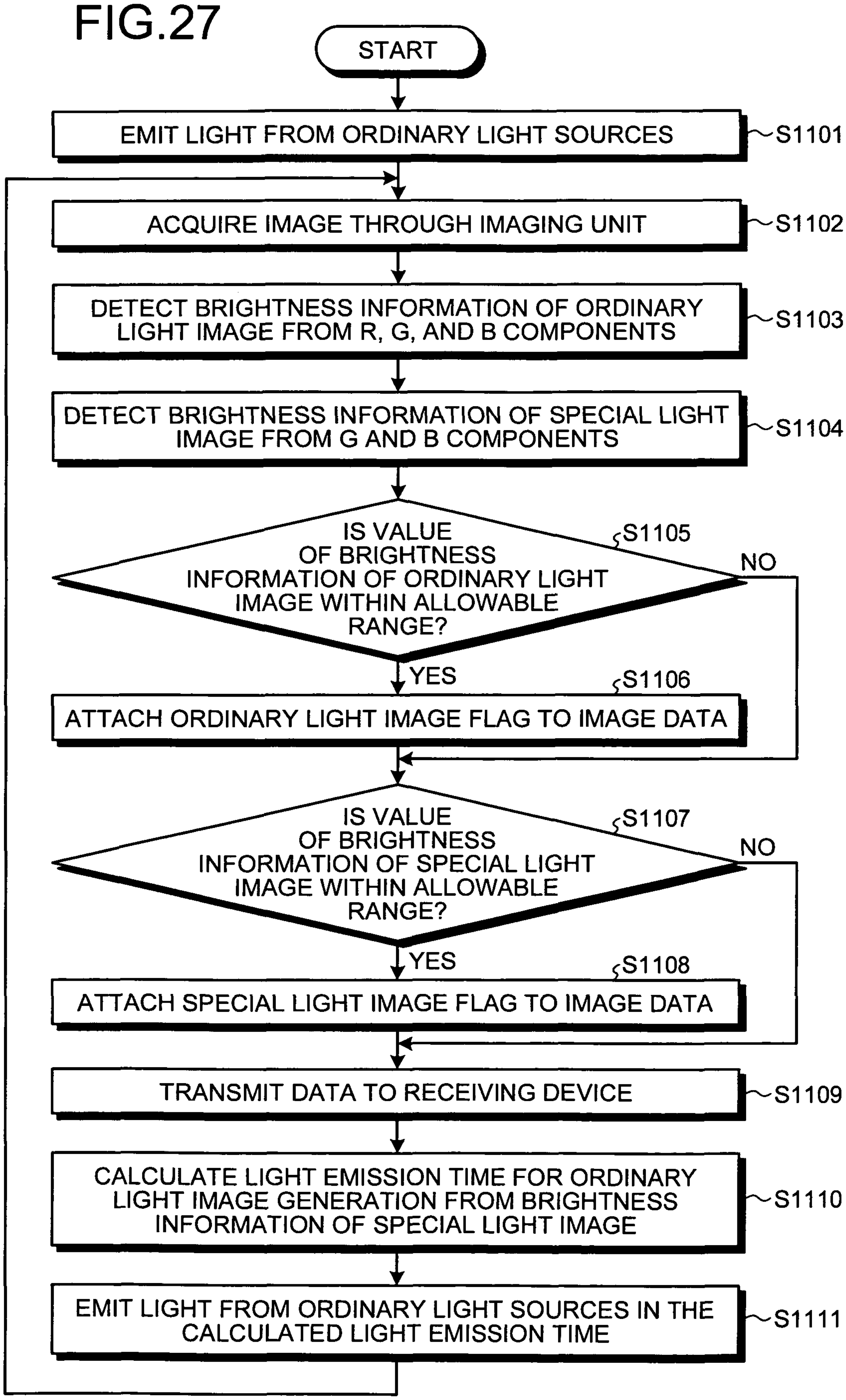
START
EMIT LIGHT FROM ORDINARY LIGHT SOURCES
S1101
ACQUIRE IMAGE THROUGH IMAGING UNIT
S1102
DETECT BRIGHTNESS INFORMATION OF ORDINARY LIGHT IMAGE FROM R, G, AND B COMPONENTS
S1103
DETECT BRIGHTNESS INFORMATION OF SPECIAL LIGHT IMAGE FROM G AND B COMPONENTS
S1104
IS VALUE OF BRIGHTNESS INFORMATION OF ORDINARY LIGHT IMAGE WITHIN ALLOWABLE RANGE?
S1105
NO
YES
S1106
ATTACH ORDINARY LIGHT IMAGE FLAG TO IMAGE DATA
IS VALUE OF BRIGHTNESS INFORMATION OF SPECIAL LIGHT IMAGE WITHIN ALLOWABLE RANGE?
S1107
NO
YES
S1108
ATTACH SPECIAL LIGHT IMAGE FLAG TO IMAGE DATA
TRANSMIT DATA TO RECEIVING DEVICE
S1109
CALCULATE LIGHT EMISSION TIME FOR ORDINARY LIGHT IMAGE GENERATION FROM BRIGHTNESS INFORMATION OF SPECIAL LIGHT IMAGE
S1110
EMIT LIGHT FROM ORDINARY LIGHT SOURCES IN THE CALCULATED LIGHT EMISSION TIME
S1111

FIG.28

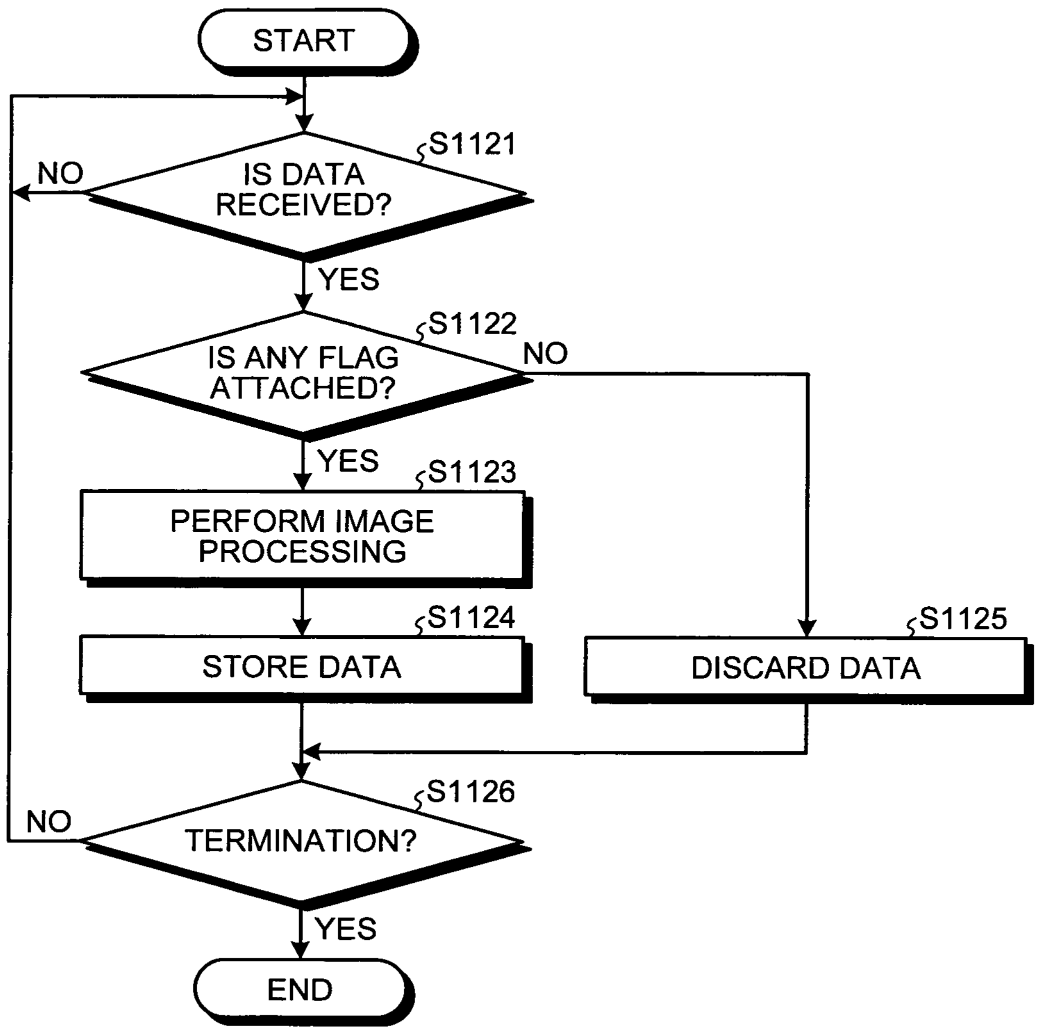
START
S1121
IS DATA RECEIVED?
NO
YES
S1122
IS ANY FLAG ATTACHED?
NO
YES
S1123
PERFORM IMAGE PROCESSING
S1124
STORE DATA
S1125
DISCARD DATA
S1126
TERMINATION?
NO
YES
END

FIG.29

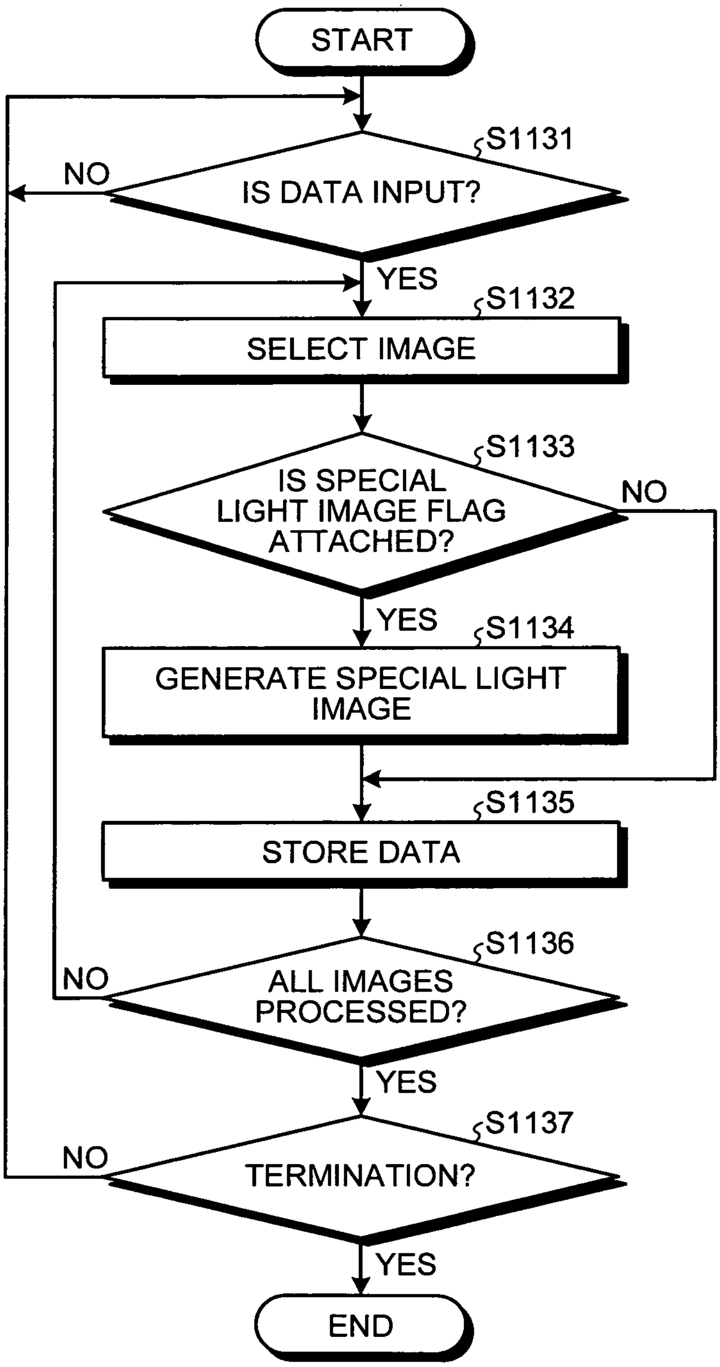
START
S1131
IS DATA INPUT?
NO
YES
S1132
SELECT IMAGE
S1133
IS SPECIAL LIGHT IMAGE FLAG ATTACHED?
NO
YES
S1134
GENERATE SPECIAL LIGHT IMAGE
S1135
STORE DATA
S1136
ALL IMAGES PROCESSED?
NO
YES
S1137
TERMINATION?
NO
YES
END

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2007215927 A